# EUROPEAN PATENT SPECIFICATION

(11) **EP 4 035 696 B1**
(45) Date of publication and mention of the grant of the patent: **03.09.2025**
(21) Application number: 22153541.2
(22) Date of filing: 26.01.2022
(51) Int. Cl.: A61L 9/015, A61L 9/20, F24F 8/22, F24F 8/26

(54) **STERILIZER AND STERILIZING METHOD**
STERILISATOR UND STERILISATIONSVERFAHREN
STÉRILISATEUR ET MÉTHODE DE STÉRILISATION

(30) Priority: 27.01.2021 PL 43678321
(43) Date of publication of application: 03.08.2022
(73) Proprietor: MILOO-ELECTRONICS Spolka z ograniczona odpowiedzialnoscia, 32-720 Nowy Wisnicz (PL)
(72) Inventor: Wlodarczyk, Milosz, 32-700 Bochnia (PL)
(74) Representative: Hudy, Ludwik

(56) References cited:
- CN-A- 111 237 917
- US-A1- 2010 178 196
- US-A1- 2019 240 370

## Description

According to the preamble of claim 1, the present invention relates to a sterilizer for filtration and disinfection of volatile medium in enclosed spaces, which also leads to disinfection of surfaces in enclosed spaces, including surfaces of objects placed in these rooms, in particular by carrying out filtration of the volatile medium in the room, disinfection of the volatile medium and sterilization of the room with ozone.

The aforementioned sterilizer for filtration and disinfection of the volatile medium comprises a disinfecting space for filtration and disinfection of the volatile medium, through which the volatile medium flows and which is surrounded by a housing, in which there is an inlet and an outlet for filtered and disinfected volatile medium connected to the disinfecting space, as well as a supply system, a forcing device of volatile medium flow and a control system for controlling the filtration and disinfection of the volatile medium flowing through the filtration and disinfection space of the volatile medium, wherein in the disinfection space of the volatile medium there is at least one ozonation channel, each with at least one ozonation chamber with at least one ozone generator, and at least one exposure channel, each provided with at least one exposure chamber with at least one UV-C radiator.

Moreover, according to the preamble of claim 12, the present invention relates to a method for filtration and disinfection of volatile medium in enclosed spaces by means of a sterilizer for filtration and disinfection of volatile medium that comprises a disinfection space for filtration and disinfection of the volatile medium, through which the volatile medium flows and which is surrounded by a housing, in which there is an inlet and an outlet of the filtered and disinfected volatile medium connected to the disinfecting space, as well as a supply system, a forcing device of volatile medium flow and a control system for controlling the filtration and disinfection of the volatile medium flowing through the filtration and disinfection space of the volatile medium, wherein in the disinfection space of the volatile medium there is at least one ozonation channel, each with at least one ozonation chamber with at least one ozone generator, and at least one exposure channel, each with at least one exposure chamber with at least one UV-C radiator.

Various types of air purification devices, disinfection devices, and ozonation devices for cleaning and disinfecting, and sterilizing air or surfaces are known. Typically, these devices are manufactured as stand-alone units, capable of performing only one primary function for which they were designed. Some of them, in particular ozonation devices, require qualified personnel equipped with measuring devices to carry out effective and safe ozonation.

Thus, from publication US6824693B1 of the patent description titled "Ozone generator and germicidal device using an ultraviolet lamp" a device is known, in which an ozone generation process for disinfecting rooms or liquids and an air purification process by UV-C radiation of a suitable wavelength occurs simultaneously. Both processes take place with the use of UV-C lamps.

From publication US20080310992A1 of the patent description titled "Apparatus and method for using ozone as a disinfectant" a device with ozone generators and UV-C radiators is known.

In turn, publication CN102397577A of the patent description titled "Method for producing ozone - generation sterilizing green environment-friendly LED energy-saving lamp" presents production of ozone with the use of a LED lamp. The LED lamp described in the aforementioned publication has no lighting function and its sole purpose is to generate ozone.

Publication CN205193531U of the patent description titled "Plate-type ozone generator's operational environment state detects and warning protection system" discloses a plate-type ozone generator system with a generator malfunction detection and a warning system. The system consists of a control system and an unspecified warning device.

Publication CN207182626U of the patent description titled "Ozone pollution voice alarm device" presents a device for detecting ozone air pollution and signaling the hazard, by means of an acoustic signal, equipped with a controller.

In addition, publication CN20483043U of the patent description titled "Intelligent air purifier" reveals an intelligent air purifier equipped with a controller, sensors, UV-C fluorescent lamps and ozone generators.

Another Chinese publication CN 111 237 917 A of the patent application description titled "Dynamic and static combined type air intelligent disinfection purification equipment" discloses dynamic and static combined type air intelligent disinfection purification equipment which comprises a housing that has an air inlet and outlet. In the housing is arranged a purification assembly, an air inducing unit, a disinfection sterilization unit and a control mechanism. According to the dynamic and static combined type air intelligent disinfection purification equipment, dynamic and static disinfection modes of air can be realized. The air can be purified while dynamic disinfection is achieved through the purification assembly, and the air quality in the closed environment can be greatly improved.

Publication US 2010/178196 of the patent application description titled "Sterilizer" presents a bio air sterilization system and method of use thereof suitable for removing and rendering harmful contaminants and particulates, such as bacteria, viruses, and molds, from air within an enclosed area. The sterilization system includes a self contained, mobile sterilization unit that includes at least an ultraviolet array, an air flow control mechanism for diverting the air flow within the system through either a filter or through an ozone removal zone to the surrounding environment.

In turn, publication US 2019/240370 of the patent application description titled "Air treatment system and method of use" teaches a built-in apparatus and method for treating air including a housing with an air inlet and an air outlet. An air mover positioned near the air outlet is configured to draw the air through the air inlet. The housing encloses an air treatment zone, such as including an oxidizing zone, and an ozone removal zone positioned downstream of the air treatment zone and oxidizing zone. The air treatment zone includes UV light and/or ozone that partially oxidizes the chemical contaminants in the air treatment zone. A UV bulb that may or may not generate ozone is positioned within or downstream to assist catalyst oxidation and/or self-clean the apparatus.

The above described air sterilization apparatus have complicated construction that is suitable for one type of sterilizer for filtration and disinfection of volatile medium.

The purpose of this invention is to provide a sterilizer for filtration and disinfection of volatile medium that comprises a disinfecting space for filtration and disinfection of the volatile medium and that has such a construction that allows providing filtration and disinfection of the volatile medium independently each other or together using the sterilizer and resulting in filtration and disinfection of the volatile medium in a room in which the sterilizer is placed or to which the volatile medium is fed as well as sterilizing this room by ozone.

According to the invention, the aim of the invention is reached by a sterilizer for filtration and disinfection of volatile medium having features of claim 1 as well as by a method for filtration and disinfection of volatile medium in enclosed spaces using the sterilizer for filtration and disinfection of volatile medium having features of claim 12. The preferred embodiments of the invention are specified in the dependent claims.

A basic idea of the invention is that the sterilizer for filtration and disinfection of volatile medium has a disinfection space for filtration and disinfection of the volatile medium, through which the volatile medium flows and which is surrounded by a housing in which there is an inlet and an outlet of the filtered and disinfected volatile medium connected to the disinfecting space, as well as a supply system, a forcing device of volatile medium flow and a control system for controlling the filtration and disinfection of the volatile medium flowing through the filtration and disinfection space of the volatile medium, wherein in the disinfection space of the volatile medium there is at least one ozonation channel, each with at least one ozonation chamber with at least one ozone generator, and at least one exposure channel, each with at least one exposure chamber with at least one UV-C radiator. The sterilizer for filtration and disinfection of volatile medium according to the invention is characterized in that a cross-sectional area of the ozonation channel ranges from 250 cm² to 500 cm² and a cross-sectional area of the exposure channel situated downstream of the ozonation channel in the flow direction of the volatile medium is from 250 cm² up to 600 cm², whereas the control system for controlling the filtration and disinfection of the volatile medium comprises a unit for switching-on and switching-off the device for forcing the volatile medium flow, a switching-on and switching-off unit of the ozone generators and a switching-on and switching-off unit of the UV-C radiators, wherein adjacent to an outer wall of the sterilizer there is an inlet channel that has a volatile medium inlet and is situated adjacent to the ozonation channel and separated from the ozonation channel by an additional wall extending the entire width of an interior of the disinfection space, wherein the inlet channel is connected to the ozonation channel via a hole located above the ozone generator being the uppermost one and allowing the flow of the medium from the inlet channel to the ozonation channel and the unit for switching-on and switching-off the device for forcing the volatile medium flow, the switching-on and switching-off unit of the ozone generators and the switching-on and switching-off unit of the UV-C radiators operate independently of each other.

According to one of preferred embodiments a particularly efficient operation of the device according to the invention can be seen in that a hole located above the uppermost placed ozone generator and allowing the flow of volatile medium from the inlet channel to the ozonation channel is made in the additional wall or is formed by positioning of an upper edge of the additional wall below a closing wall that closes the inlet channel and the ozonation channel from above.

It is preferred that the exposure channel extends across the entire width of the interior of the disinfection space and is situated adjacent to the ozonation channel and separated from the ozonation channel by a separating wall and whereby the exposure channel is connected to the ozonation channel via a hole located in a region of the bottom wall of the sterilizer and allowing the flow of the volatile medium from the ozonation channel to the exposure channel.

Additionally, in one of preferred embodiments an opening located in the region of the bottom wall of the sterilizer and allowing the flow of the volatile medium from the ozonation channel to the exposure channel is made in the separating wall or is formed by positioning a lower edge of the separating wall above a bottom wall which closes the ozonation channel and the exposure channel from below.

In another preferred embodiment at side walls of the sterilizer there are situated posts with holes for mounting the ozone generators and limiting at least on sides the ozonation channel, whereby in a region of a lower part of the exposure channel and in a region of an upper part of the exposure channel there are sockets for embedding the UV-C radiators.

In further preferred embodiment the exposure channel is connected to an air chamber in which there is the device forcing the flow of the volatile medium through the sterilizer and which is situated adjacent to an upper filter chamber and an outlet of which is the outlet of the filtered and disinfected volatile medium covered by an outlet filter and an outlet grille or a spigot stub whereas the ozonation channel is connected to the inlet of the filtered and disinfected volatile medium, situated at a front, side or back of the sterilizer and covered by a stub pipe, to which an additional movable arm is mounted comprising a set of tubes connected to each other by articulated joints and terminated with an interchangeable tip for pulling off the filtered and disinfected volatile medium.

An another preferred development of the invention foresees that the sterilizer is a stationary central unit connected by at least one of its inlets to pull-off ducts with interchangeable tips for suction of the volatile mediumfrom one or more rooms and connected by at least one of its outlets to blowing ducts with diffusers for supplying purified volatile medium or ozone to one or more rooms.

According to a further embodiment a flexible pipe is connected to the spigot stub having an interchangeable tip placed inside a vehicle and used for supplying purified volatile medium or ozone to a vehicle interior.

In yet another embodiment according to the invention the sterilizer is one of sterilizers of a system of simultaneous operating numerous sterilizers for filtration and disinfection of the volatile medium and for surface disinfection in enclosed spaces communicating by means of a control system to each sterilizer being associated with at least one sterilizer closest to it.

A basic idea of the invention is also a method for filtration and disinfection of volatile medium in enclosed spaces using a sterilizer for filtration and disinfection of volatile medium that has a disinfection space for filtration and disinfection of the volatile medium, through which the volatile medium flows and which is surrounded by a housing in which there is an inlet and an outlet of filtered and disinfected volatile medium connected to the disinfecting space as well as a supply system, a forcing device of volatile medium flow and a control system for controlling the filtration and disinfection of the volatile medium flowing through the filtration and disinfection space of the volatile medium, wherein in the disinfection space of the volatile medium there is at least one ozonation channel, each with at least one ozonation chamber with at least one ozone generator, and at least one exposure channel, each with at least one exposure chamber with at least one UV-C radiator. The method for filtration and disinfection of volatile medium is characterized in that a process selected from a volatile medium filtration process and/or a volatile medium disinfection process is carried out by means of the sterilizer for filtration and disinfection of the volatile medium that has a cross-sectional area of the ozonation channel ranging from 250 cm² to 500 cm² and the cross-sectional area of the exposure channel situated downstream of the ozonation channel in the flow direction of the volatile medium ranging from 250 cm² up to 600 cm², and has the control system for controlling the filtration and disinfection of the volatile medium which comprises a unit for switching-on and switching-off the device for forcing the volatile medium flow, a switching-on and switching-off unit of the ozone generators and a switching-on and switching-off unit of the UV-C radiators, wherein the unit for switching-on and switching-off the device for forcing the volatile medium flow, the switching-on and switching-off unit of the ozone generators and the switching-on and switching-off unit of the UV-C radiators operate independently of each other and the volatile medium filtration process and/or the volatile medium disinfection process is selected by the control system.

After exceeding a predetermined level of ozone concentration in the volatile medium a process of ozone destruction to molecular oxygen begins by switching on the UV-C radiators and by switched-off ozone generators and is carried until the safe ozone level is reached.

Other features and advantages of the invention will become apparent from the description hereinafter with reference to the attached schematic drawings, which are provided only by way of non-limitative examples of the invention, wherein:
Fig. 1 and 2 show a sterilizer in an axonometric view from top;
Fig. 3 and 4 show the sterilizers in a front view differing in their appearance of control panel indicators and in construction;
Fig. 5 shows a longitudinal section of one embodiment of the sterilizer;
Fig. 6 shows a cross-sectional view of one embodiment of the sterilizer;
Fig. 7 and 8 show embodiments of the sterilizers in an exploded view;
Fig. 9 shows an universal post with two ozone generators after the has been removed from the housing;
Fig. 10 shows housing of sockets, which are used for embedding or mounting UV-C radiators;
Fig. 11 shows two sets of ozone generators mounted in universal posts with side rails;
Fig. 12 shows three sets of generators mounted on the same universal post;
Fig. 13 shows sets of double UV-C radiators, mounted in sockets;
Fig. 14 shows UV-C radiators, mounted individually in the sockets;
Fig. 15 and 16 show a sterilizer provided with an additional movable arm comprising a set of tubes connected to each other by articulated joints;
Fig. 17 shows a sterilizer 401, which operates as one stationary central unit providing disinfection or sterilization of more than one room at a time;
Fig. 18 shows a sterilizer, which functions as an interior ozonation device for vehicles;
Fig. 19 shows a system of simultaneous operation of multiple sterilizers in enclosed spaces with very large volumes, Fig. 20 shows a block diagram of the sterilizer;
Fig. 21 shows a user panel for operating the sterilizer;
Fig. 22 shows a graph of the ozonation process;
Fig. 23 shows a control of the generators in an "on-off" cycle;
Fig. 24A and Fig. 24B show a block diagram of an algorithm of a sterilizer operation in a filtration mode;
Fig. 25A and Fig. 25B show a block diagram of an algorithm of a sterilizer operation in an UV-C disinfection mode; and
Fig. 26A, Fig. 26B, Fig. 26C and Fig. 26D show a block diagram of an algorithm of a sterilizer operation in an ozone sterilization mode.

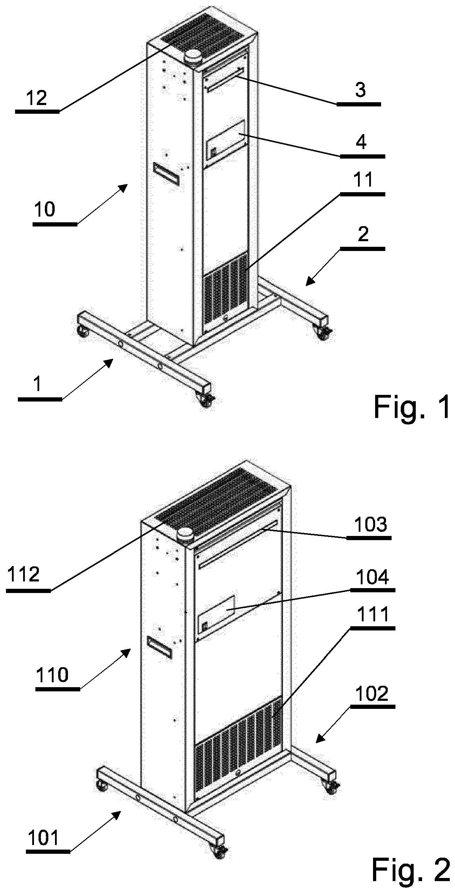

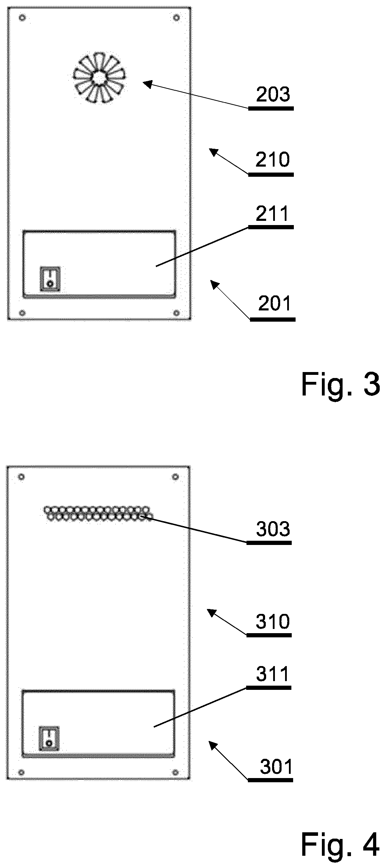

Fig. 1, 2, 3 and 4 show a sterilizer 1, 101, 201, 301, respectively, for filtration and disinfection of volatile medium, in an axonometric view, with a housing 10, 110, 210, 310 and a base 2, 102 on which the housing is mounted. Embodiments of the sterilizer differ in appearance, size, method of attachment to the base and their design as well as technical parameters. The sterilizers 1, 101, differing in size, for example, are shown in Fig. 1 and 2 in an axonometric view, obliquely from above, and the sterilizers differing in an appearance of indicators 3, 103, 203, 303 and control panels 4, 104 and in design are shown in front view in Fig. 3 i 4. The base 2, 102 of the sterilizer may be castors together with a specially designed structure, as shown in Fig. 1 and 2, wherein the castors are placed on a multi-armed structure and a fixed and immovable base of various shapes or short legs are mounted in corners of a lower part of the device housing. Other configurations of the sterilizer base not shown in the figures are also possible.

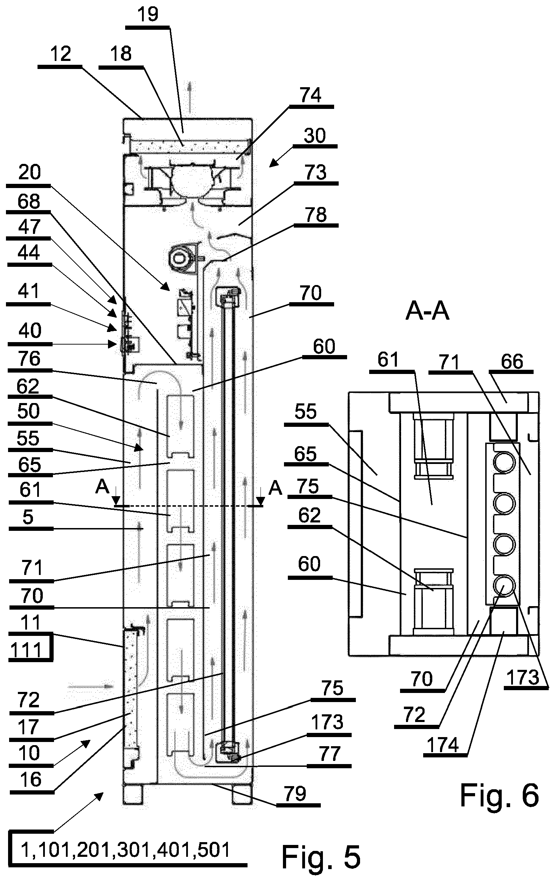

In one embodiment, shown in Fig. 5, the housing has the shape of a cuboid with the base of a rectangle or triangle or circle, or parts thereof, or a combination of parts of plane figures. In other embodiment, the housing may have a shape of a pyramid, cylinder, cone or a combination of spatial figures.

Regardless of an external appearance, size, base structure and technical parameters, each type of the sterilizer for filtration and disinfection of the volatile medium 5 comprises a space 50 for filtration and disinfection of the volatile medium through which the volatile medium 5 flows and which is surrounded by the housing of, for example, cuboidal shape 10, 110, 210, 310, 410, 510 respectively, in which there is, connected to the volatile medium filtration and disinfection space 50, an inlet opening 11, 111, 211, 311 and an outlet opening 12, 112 of the filtered and disinfected volatile medium 5, and a supply system 20, a device 30 forcing the flow of volatile medium, for example a fan, and a control system 40 for controlling the filtration and disinfection of the volatile medium flowing through the volatile medium filtration and disinfection space, wherein the volatile medium filtration and disinfection space 50 comprises at least one ozonation channel 60, each with at least one ozonation chamber 61 with at least one ozone generator 62, and at least one exposure channel 70, each with at least one exposure chamber 71 with at least one UV-C radiator 72. The internal design of the sterilizer is shown in Fig. 5.

The description of the internal design of the sterilizer and its operation will be presented in more detail with reference to the sterilizers shown in Fig. 1 and 2, however, it should be noted that the description of the internal design of the sterilizers and their operation, regardless of their external appearance, size, base construction and technical parameters, will be the same unless expressly stated.

In the embodiment shown in Fig. 5, the housing has the shape of a cuboid with a rectangular cross-section and is made of sheet metal or plastic. Taking into consideration the simplicity of the construction, the internal space is divided by internal walls into connecting channels, which connect to each other, or into closed rectangular internal spaces for the sterilizer components, usually with flat sheets or flat sheet metal or plastic elements or profiles, for example channels or angles, or elements with L-shaped, C-shaped, U-shaped cross-sections or combinations thereof. The channels formed inside have cross-sections adapted to the shapes of the components constructed or placed in them, but the most common shape of their cross-section is a rectangle.

Thus, the cross-sectional area of the ozonation channel 60 in one embodiment is 250 cm², and in other embodiment it is 500 cm², and in still another embodiments the cross-sectional area of the ozonation channel 60, depending on the size, is selected from the range of 250 cm² to 500 cm². In contrast, the cross-sectional area of the exposure channel 70 located in the direction of the flow of the volatile medium 5 downstream of the ozonation channel 60 is 250 cm² in one embodiment and 600 cm² in other embodiment, and in still another embodiment the cross-sectional area of the exposure channel 70 according to the size is selected from the range of 250 cm² to 600 cm². Both, the ozonation channel 60 and the exposure channel 70, have a rectangular cross-section and are large enough to allow, after installation of ozone generators or UV-C radiators according to the function of the channel, a free flow of the volatile medium through each of the channels. Other cross-sectional shapes of said channels are also provided in order to accommodate components such as ozone generators and UV-C radiators.

In embodiments not shown in the drawings, the sterilizers having the volatile medium inlet positioned above the uppermost ozone generator or at the height of the uppermost ozone generator, have a transverse dimension of the ozonation channel, measured from a front wall of the sterilizer to a wall separating the ozonation channel from the exposure channel, larger or greater than the transverse dimension of the exposure channel from 2 cm to 10 cm.

In the example shown in Fig. 5, where the inlet 11, 111 of the volatile medium 5, passing through a grille 16 and a prefilter 17, is located at the bottom of the sterilizer 1, 101, the sterilizers additionally have an inlet channel 55 located adjacent an outer wall of the sterilizer, which allows the volatile medium 5 to flow into an inlet of the ozonation channel 60 located above the uppermost ozone generator 62.

The ozonation channel 60, separated from the inlet channel 55 by an additional wall 65 extending over the entire width of the interior of the disinfection space 50, is separated from the exposure channel 70 by a separating wall 75, which has a through hole or, with its upper edge, is positioned below a closing wall 68, which closes the inlet channel 55 and the ozonation channel 60 from above, whereby an upper through hole 76 is formed at the top connecting the inlet channel 55 and the ozonation channel 60. The inlet channel 55 has a cross-sectional area equal to or up to 25% smaller than the cross-sectional area of the ozonation channel, since in many embodiments no devices nor components of the sterilizer are mounted in the inlet channel. The separating wall 75 can either be removable or be permanently fixed to outer walls of the sterilizer housing.

In an upper region of the sterilizer there is the control system 40 with a switching-on unit 41 of the device for forcing the volatile medium flow, a switching-on unit 44 of the ozone generators 62 and a switching-on unit 47 of the UV-C radiators 72, which supervises the filtration and disinfection process.

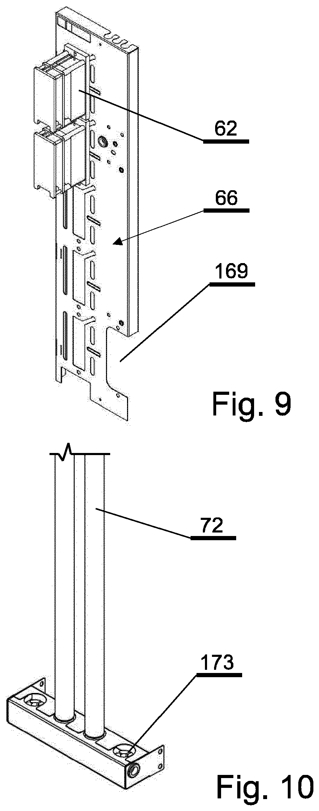

Furthermore, the ozonation channel 60 is separated from the exposure channel 70 by the separating wall 75 extending over the entire width of the interior of the disinfection space 50. The separating wall 75 has a bottom opening or side opening or its bottom edge is positioned above a bottom wall 79 of the sterilizer housing, so that a bottom through hole 77 is formed, connecting the ozonation channel 60 and the exposure channel 70 at the bottom. The separating wall 75 can either be removable or be permanently fixed to the outer walls of the sterilizer housing or to universal posts 66, shown in more detail in Fig. 9, which are for mounting the plate ozone generators 62 by means of holes made in the universal posts. The universal posts 66 may be one piece or multi-piece. One universal post in one embodiment allows for installation of up to five ozone generators. Mounting the ozone generators on the post consists of screwing them on with two lenticular screws and using cable grommets for the electrical wires coming out of the ozone generators.

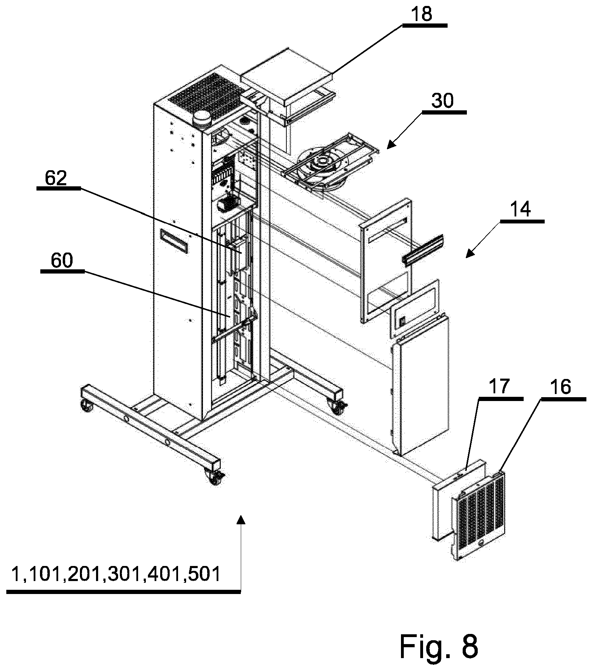

The exposure channel 70 is connected to an air chamber 73, in which there is the device 30 forcing the flow of the volatile medium through the sterilizer, and adjacent thereto there is an upper filter chamber 74, the outlet thereof is the outlet 12 of the filtered and disinfected volatile medium 5, covered by an outlet filter 18 and an outlet grille 19, as shown in more detail in Fig. 5 and Fig. 8. The flow of the volatile medium 5 between the exposure channel 70 and the air chamber 73 is directed by means of a bend 78 of the separating wall 75 and possibly additional elements redirecting the flow of the volatile medium 5. In the region of a lower part of the exposure channel 70 and in the region of an upper part of the exposure channel 70, brackets 174 of a socket 173 housing are provided for fixing them.

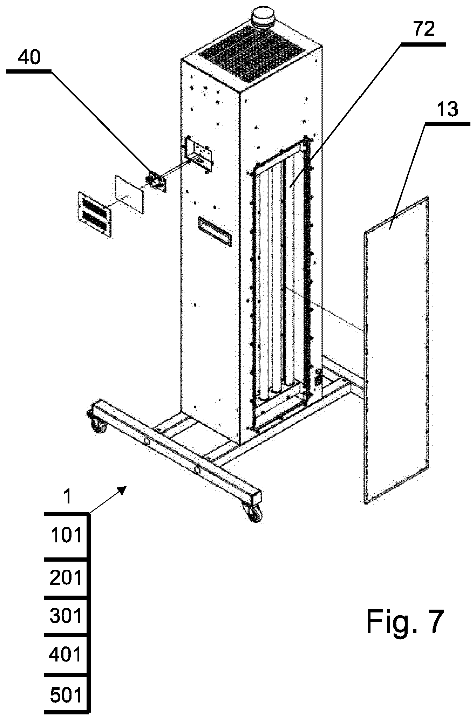

As shown in Fig. 7, access to the UV-C radiators 72, and hence their installation and replacement, is enabled by a removable cover 13, covering the opening and fastened to a rear outer wall of the sterilizer housing 10, 110, 210. Access to components of the control system 40 for controlling the filtration and disinfection of the volatile medium, in the embodiment shown in Fig. 7, is possible after they have been removed from the housing 10, 110, 210.

In turn, access to other components of the sterilizer 1, 101, 201, 310, 401, 501, including the ozone generators 62, is possible after disassembly of a front cover of a front wall 14 of the outer housing 10, 110, 210 of the sterilizer, as shown in Fig. 8. The front cover of the front wall 14 of the outer housing 10, 110, 210 of the sterilizer in one embodiment that is not shown, is one-piece. In other embodiment, the front wall 14 is divided as shown in Fig. 8.

Fig. 9 shows the aforementioned universal post 66 with two ozone generators 62 after it has been removed from the housing. The universal post 66 has holes, by means of which the ozone generators 62 are mounted, and a mounting hole 169. In one embodiment, the sterilizer may not have any ozone generator and become a device solely for cleaning and disinfecting the volatile medium.

In turn, Fig. 10 shows the housing of sockets 173, which are positioned in the region of the lower part of the exposure channel 70 and in the region of the upper part of the exposure channel 70, and which are used for embedding or mounting the UV-C radiators 72 therein. In the embodiment shown in Fig. 10, the housing of sockets 173 is adapted to accommodate up to four UV-C radiators. The housing comprises a rail in which the sockets of the UV-C radiators are located. The housing is furthermore provided with cable grommets. Sockets of various models are adapted to accommodate UV-C radiators of various sizes and with a different number of pins. At the top, the UV-C radiators are mounted in the same housings. In one embodiment, it is possible not to use a single UV-C radiator in the sterilizer, so that the sterilizer does not have the function of disinfecting with UV-C radiators.

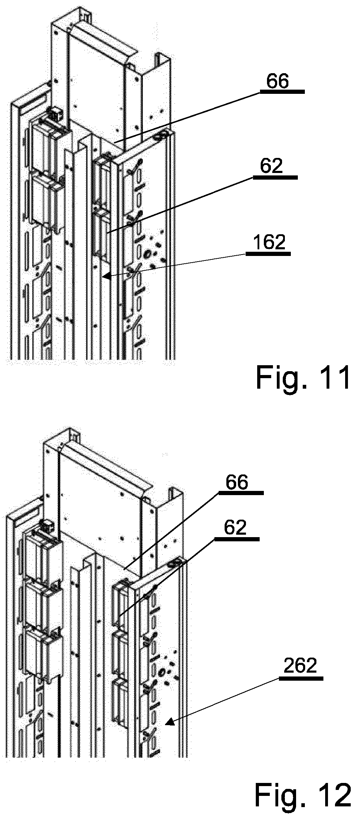

In Fig. 11, there are shown two sets 162 of the ozone generators 62, mounted in the universal posts 66 with side rails 67, and in Fig. 12, there are shown three sets 262 of the ozone generators 62, mounted in the same universal post 66 with the side rails 67.

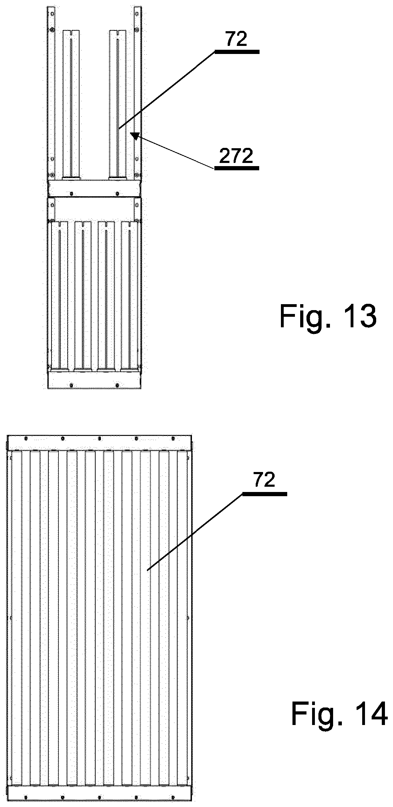

In Fig. 13, there are shown sets 272 of double UV-C radiators 72, mounted in sockets, and in Fig. 14, there are shown the UV-C radiators 72, mounted individually in the sockets.

The sterilizer shown in Fig. 5 and Fig. 8 has the air chamber 73, with which the upper filter chamber 74 is adjacent, while in other embodiment, not shown in the figures, the sterilizer has either a side filter chamber or a rear filter chamber, with which it is adjacent and connected to the air chamber in which the device for forcing the volatile medium through the sterilizer is located. An outlet of the side filter chamber or the rear filter chamber is a side outlet or a rear outlet of the filtered and disinfected volatile medium 5, which in one embodiment may be the only outlet of the filtered and disinfected volatile medium 5, whereby another outlet, for example the upper outlet 12, may be blinded or may not be provided. In another embodiment, the rear outlet or side outlet may be an additional outlet for the filtered and disinfected volatile medium 5.

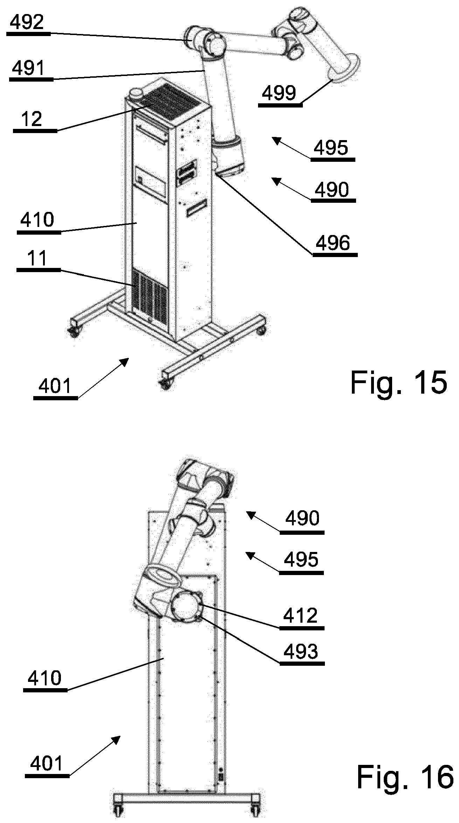

A similar situation may apply to the inlet 11 of the filtered and disinfected volatile medium 5, which may be located not only at a front of the sterilizer, but also at a side or rear of the sterilizer, which is shown in Fig. 15 and Fig. 16. In the embodiment shown in Fig. 15 and Fig. 16, the sterilizer 401 is provided with an additional movable arm 490 comprising a set 495 of tubes 491 connected to each other by articulated joints 492 and for extracting the filtered and disinfected volatile medium 5. At one end, the set 495 of the tubes 491 has a stub pipe 496 attached to either a sole or an additional inlet grille 493 of the volatile medium to be treated, and at the other end of the set 495 of the tubes there are interchangeable tips 499, of different shapes and cross-sections, fitted as required. The movable arm 490 enables the extraction of the volatile medium from almost anywhere in the room that is within the range of the device. Thanks to this, it is possible to suck in pollutants in the air directly from the source of their emission. For example, the source of an emission of pollutants in a dentist's office is most often located near the chair on which the procedure is carried out. For example, the polluted air is prefiltered through a pre-filter, disinfected with UV-C radiation and re-filtered through an activated carbon filter. Alternatively, an anti-smog filter can be used instead of the activated carbon filter, which is even more effective in cleaning the air from solid particles. After cleaning and disinfecting, the volatile medium is then blown back into the surgery room. Owing to this solution, it is possible to clean the air from solid particles, remove unpleasant smells, but above all, it is possible to effectively destroy micro-organisms contained in the volatile medium, for example in the air. Air disinfection in the dentist's office through the use of the movable arm that allows air extraction from any location is even more effective as the greatest amount of microorganisms is generated during and in the place where the dental procedure is carried out. These can be bacteria, viruses, fungi and other microorganisms that pose a risk to human health. Although such a solution is dedicated to the dental offices, if necessary, it can also be used in other places where there are local sources of emission of pollutants and microorganisms.

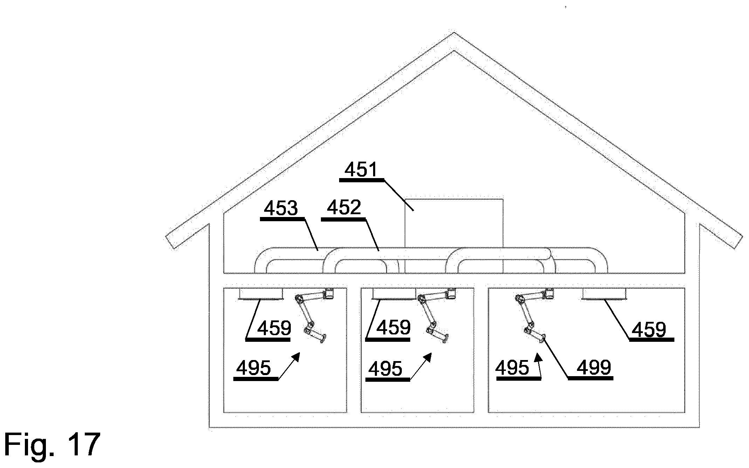

Fig. 17 shows the sterilizer 401 for filtration and disinfection of the volatile medium 5 and disinfection of surfaces in closed spaces, which operates as one stationary central unit 451 providing disinfection or sterilization of more than one room at a time. The sterilizer 401 operating in such a system has special pull-off ducts 452 and blowing ducts 453 fixed to the housing of the sterilizer 401. The sterilizer 401 in the embodiment shown in Fig. 17, together with the system of pull-off ducts 452 and blowing ducts 453, as well as extraction units with set 495 of interchangeable tips 499 and diffusers 459, is able to extract air from one or more rooms, clean and disinfect it, and then blow back into these rooms. It is also possible to extract air from the rooms, which is then used for ozone production in the sterilizer and to supply ozone to other rooms. Owing to the use of the diffusers that evenly supply ozone to the rooms, the substance is very evenly distributed in each room. The diffusers and extractors are equipped with dampers controlled by the control system to which data are sent from sensors, especially ozone sensors located in rooms and communicating wirelessly with the central sterilizer through the control system, thanks to which it is possible to precisely control the entire process, including making decision on which rooms the air is extracted from and to which it is blown. By means of the control system it is possible to control the efficiency of air flow to each room.

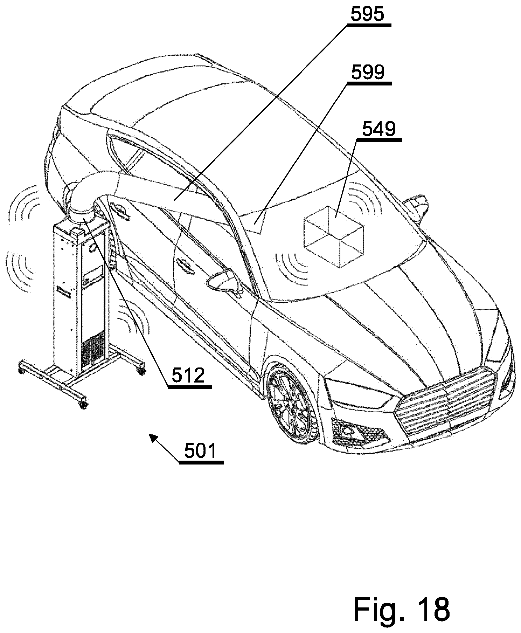

In Fig. 18, the sterilizer 501 is shown, which functions as an interior ozonation device for vehicles. To this end, the sterilizer has a spigot stub 512 in place of an exhaust grille, to which a flexible pipe 595 is mounted, an interchangeable tip 599 is placed inside a vehicle and used for supplying purified volatile medium or ozone to a vehicle interior removable tip 599 thereof is inserted inside the vehicle through an opening created by a tilted glass pane. The even distribution of ozone in the interior is ensured by the correct positioning of the tip of the flexible pipe 595 inside the car, switching on the fans in the car and switching on the internal air circulation. An ozone sensor 549 is placed inside the car, communicating wirelessly with the sterilizer controller. The uniform distribution of ozone in the interior is ensured by the appropriate positioning of the tip of the flexible pipe 595 inside the car, switching on the fans in the car and turning on the internal air circulation. Inside the car is an ozone sensor 549 that communicates wirelessly with the sterilizer controller. This makes it possible to control the ozone concentration in the car and to determine the required sterilization time in automatic mode.

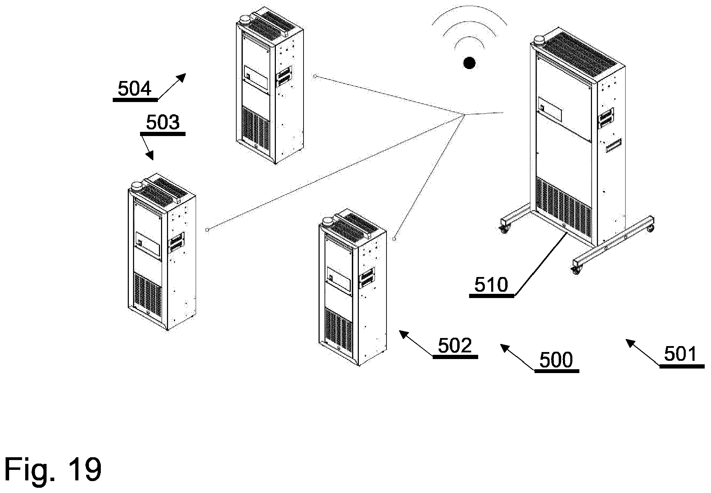

Fig. 19 shows a system 500 of simultaneous operation of multiple sterilizers 501, 502, 503, 504 for filtration and disinfection of the volatile medium and disinfection of surfaces in enclosed spaces with very large volumes, where it is impossible to carry out the process with one sterilizer or where one larger device can be replaced by several smaller ones. In one of the sterilizer operating systems shown in Fig. 19, all working sterilizers are equivalent, that is to say, no master device or slave devices can be distinguished. The operation of the system consists in arranging the devices within a disinfected room in such a way that each of the sterilizers 501, 502, 503, 504 is responsible for its space of the room and that the entire room is within the operating range of at least one sterilizer. The entire cycle of sterilizers operation in the system of parallel operation consists of several stages. After setting up the sterilizers, they are communicated with each other by means of a control system in such a way that one sterilizer is linked to the devices closest to it and on the assumption that each sterilizer is communicated with at least one another sterilizer, but the most often each sterilizer is communicated with several neighboring sterilizers.

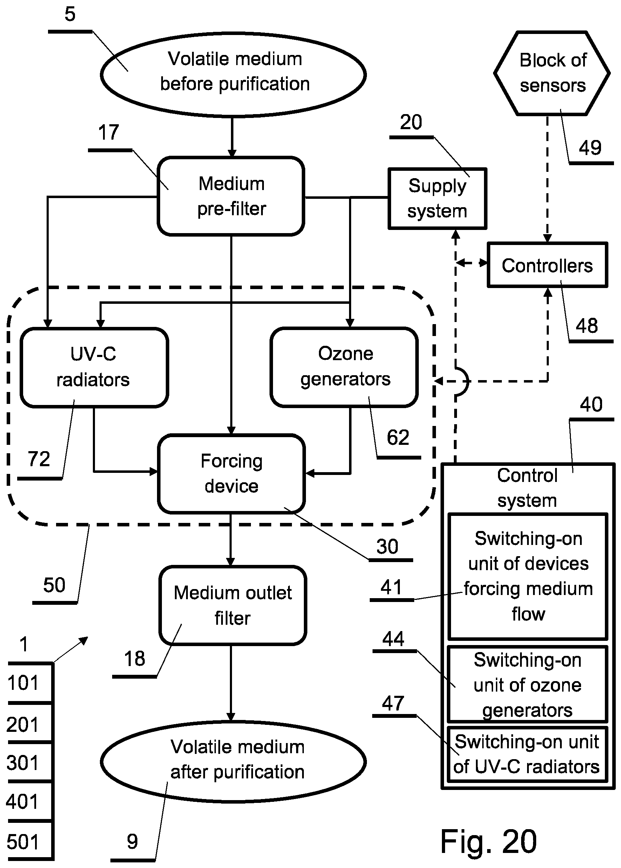

Fig. 20 shows a block diagram of the sterilizer 1, 101, 201, 301, 401, 501, through the inlet of which the volatile medium 5 is sucked in before purification and through the outlet of which a volatile medium 9 is blown out after purification, in the filtration and disinfection process supervised by the control system 40 with the switching-on unit 41 of the device for forcing the volatile medium flow, the switching-on unit 44 of the ozone generators 62 and the switching-on unit 47 of the UV-C radiators, thanks to controllers 48 and a block 49 of sensors.

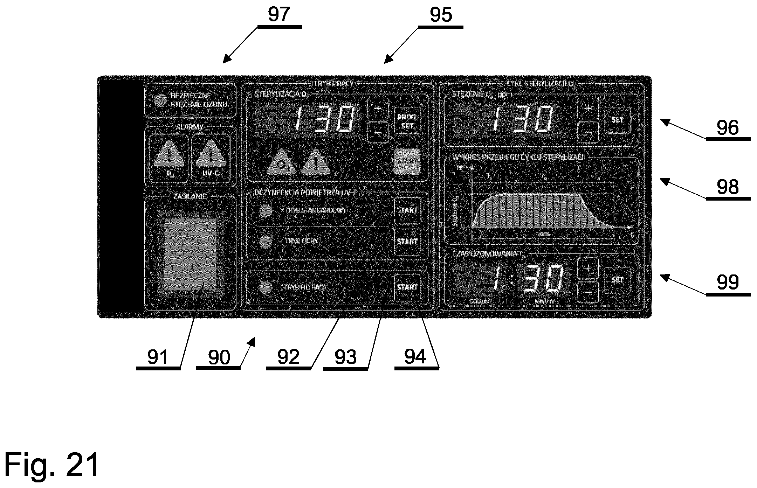

Fig. 21 shows a user panel 90 for operating the sterilizer. The user panel 90 has a main switch 91, LEDs 97 for warning, failure and safe ozone concentration, function buttons 92, 93, 94, 95, 96 and 99 and digital displays 98. By using the user panel 90, it is possible to start and stop each operating mode, setting configuration parameters and viewing working time counters. The panel also presents various messages, such as a current operating mode, parameter codes, currently running additional functions, current concentration, ozonation time and others, and alarms. The user panel 90 is also equipped with a lock function making it impossible to execute any command except to unlock it again. Turning the main switch 91 to the "O" position puts the device in sleep mode. This means that the current ozone concentration is monitored even though the user panel 90 is switched off.

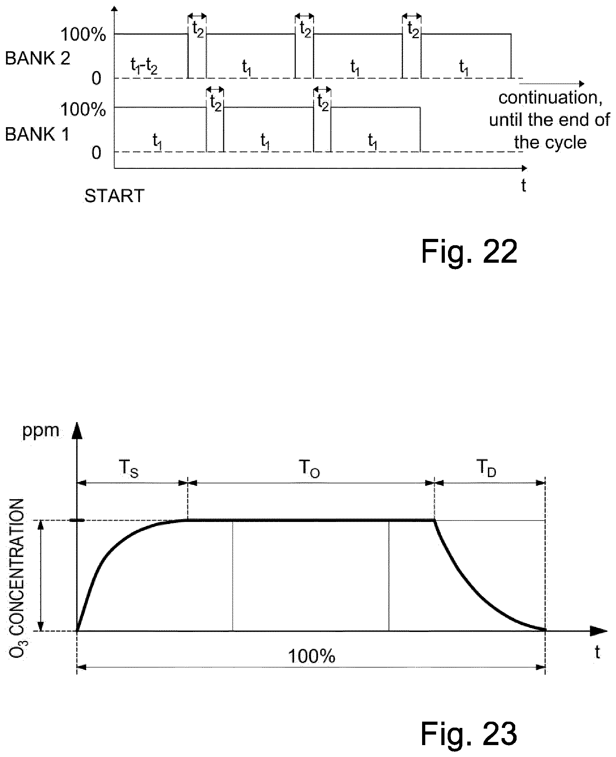

Fig. 22 shows a graph of the ozonation process. The ozonation cycle, as shown in Fig. 22, is divided into three phases. In the start-up phase T_{S}, the ozone generators and fans are started at the highest efficiency. The ozone concentration increases until the operating level is reached. In the ozonation phase T_{O}, this ozone concentration is stabilized at the working level defined by configuration parameters and depends on the selected operation mode. During this phase, the generators are controlled in an "on-off" cycle, as shown in Fig. 23, in order to stabilize a given ozone concentration. The ozonation time T_{O} is determined dynamically by the controller during the device operation on the basis of the required ozonation time as a function of the working ozone concentration.

During the ozone destruction phase T_{D}, which follows the end of the ozonation phase, the generators are switched off and UV-C radiators are turned on, which emit UV-C radiation of a specific wavelength, which accelerates the decomposition of ozone molecules (O₃) to molecular oxygen (O₂). The destruction phase can also be started after emergencies related to the sterilization process. During the entire ozonation cycle, two ozone generator banks are used: BANK 1 and BANK 2. The division into two banks of the ozone generators always occurs, regardless of the number of the ozone generators in the device. At the beginning of the start-up phase, both banks are started simultaneously, after which they are controlled according to the scheme shown in Fig. 23. BANK 2 operates for the time specified as the difference "t₁-t₂". After this time, it is switched off for a standstill period t₂ and switched on again. The "on-off" cycle repeats with a period t₁ until the end of the ozonation process. BANK 1 operates for the time defined as t₁. After this time has elapsed, it is switched off for the standstill period t₂ and switched on again. The "on-off" cycle repeats with the period t₁ until the end of the sterilization process. Between the switching off of BANK 1 and the switching on of BANK 2, there is a constant pause of a few seconds, the so-called dead time, not shown in Fig. 23.

The generated ozone as a gas reaches all the places in the room intended for the ozonation, after proper preparation of the room and assuming that the sterilizer with the right capacity for the room has been chosen, and thus neutralizing pathogens. When ozonation is completed, there is a transition to the so-called ozone destruction phase. Destruction of ozone continues until the ozone concentration in the room is 0.1 ppm, which is safe for the user. The operation of the sterilizer in the ozonation mode is signaled by the sterilizer in a characteristic and visible way. During the entire ozonation process, the warning signal light, located on the top of the device, and a piezoelectric sound signal work. The active sterilization process is further indicated by the user panel 90, shown in Fig. 21, and its last phase, the so-called ozone destruction, is signaled by the active illuminated operating panel of the UV-C sources. As soon as the ozonation is completed and the safe concentration of ozone is reached, the "Safe concentration of ozone" indicator light is illuminated.

Due to the properties of ozone that are harmful to human and animal health and life, the sterilizers have been equipped with ozone sensors, which monitor its concentration on an ongoing basis, as well as a number of safeguards preventing the possible effects of exposure to high ozone concentration. If an ozone concentration of 10 ppm is exceeded during the ozonation process, the process will be stopped immediately and ozone destruction will begin, ending when the safe concentration is reached. The ozone sensor is located inside the sterilizer, at its side part, right under the fan and away from the ozone generators, and is also protected by a filter mat, thanks to which its indications correctly determine the ozone concentration that is in the room. The monitoring of the current ozone concentration has two main purposes: it allows to determine the time of the ozonation at a given concentration so that it is effective and it has a safety function, since errors and messages of the ozonation process are determined from it. The ozone sensors in the sterilizers are calibrated by reading the sensor value, among other things, at a concentration of 0 ppm and at several other measuring points.

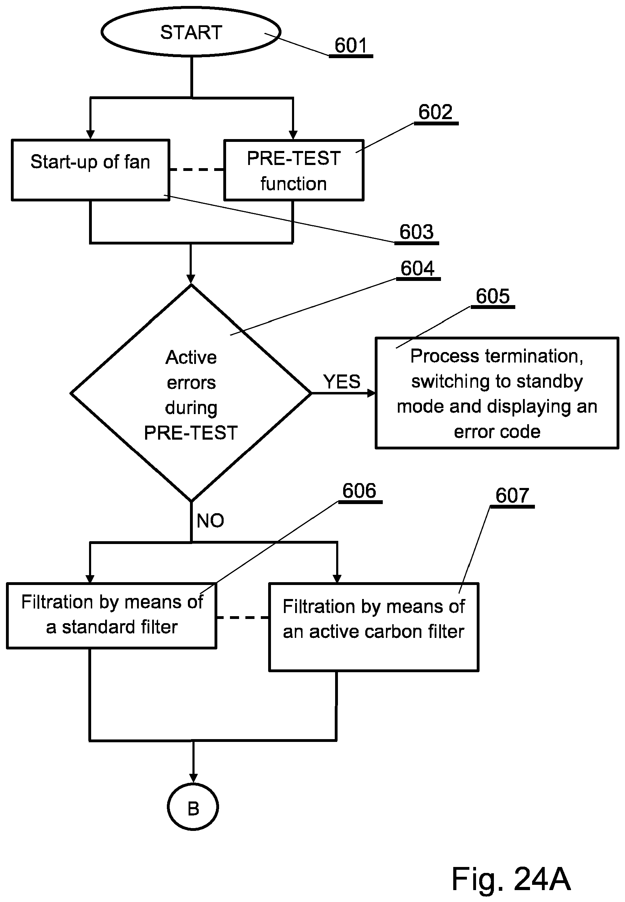

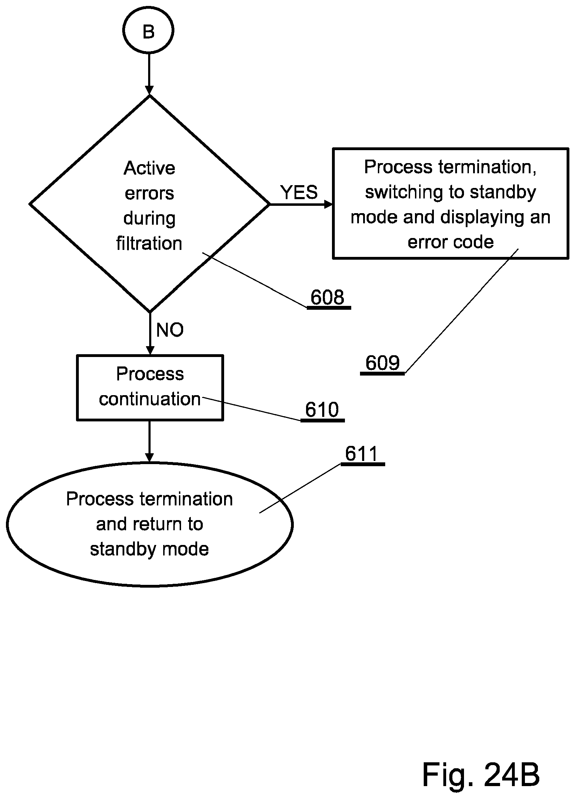

Fig. 24A and Fig. 24B show a block diagram of an algorithm of a sterilizer operation in a filtration mode. When the sterilizer is switched on in step 601, a PRE-TEST function starts in step 602, during which a fan in step 603 is start-up or activated and its correct operation is checked. The PRE-TEST function may or may not show a fan error in step 604. If the error is detected, the start-up process is terminated in step 605 and the sterilizer returns to standby mode and displays an error code. If no errors are detected, the device starts working to operate in filtration mode. Filtration takes place by means of a standard filter in step 606 and/or by means of an activated carbon filter in step 607. During filtration, the fan continues to be subjected to correct operation monitoring in step 608. If the error is detected, the process is terminated and the standby mode is entered, and an error code is displayed in step 609. No errors mean the process continues in step 610. The filtration process can be terminated by the user at any time. When the process in step 611 is terminated, the device returns to the standby mode.

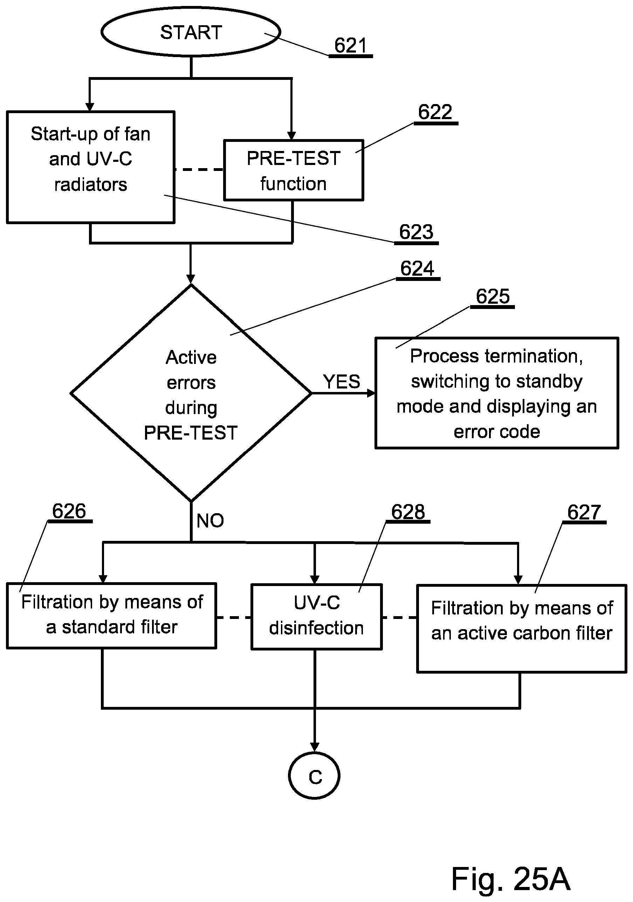

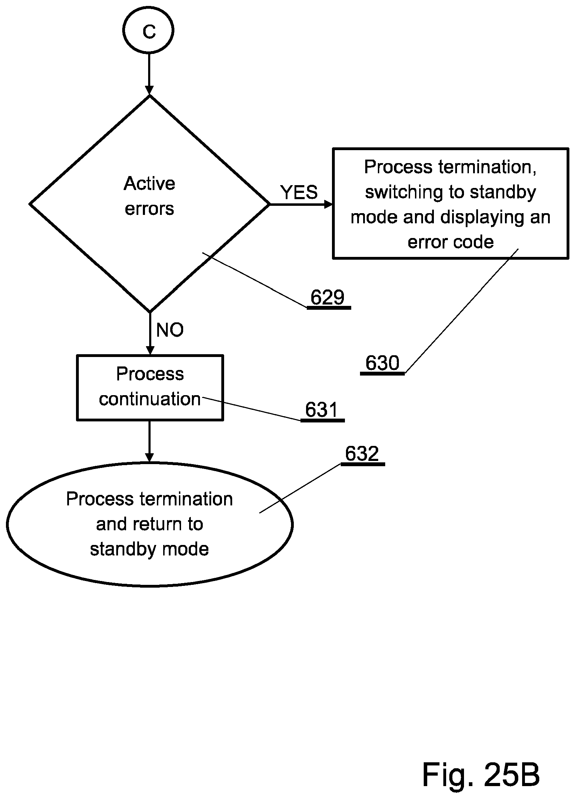

Fig. 25A and Fig. 25B show a block diagram of an algorithm of a sterilizer operation in an UV-C disinfection mode. After switching on the sterilizer in step 621, a PRE-TEST function in step 622 starts, during which a fan and UV-C radiators are all activated in step 623, the correct operation of which is checked. The PRE-TEST function may or may not show an error of the fan and/or UV-C radiators in step 624. If an error is shown, the process is terminated and the device returns to standby mode and displays a corresponding error code in step 625. If no errors are indicated, the sterilizer starts in the UV-C disinfection mode. During disinfection mode the air is cleaned by two filters in steps 626, 627 and is disinfected by UV-C radiators in step 628. During disinfection, the fan and UV-C radiators are monitored for correct operation in step 629. Detection of any error terminates the process, conditions the return to standby mode and display of error codes in step 630. The UV-C disinfection process can be terminated by the user at any time. No errors mean the process continues in step 631. The process is terminated by the user at any time. When the process in step 632 is terminated, the device returns to the standby mode.

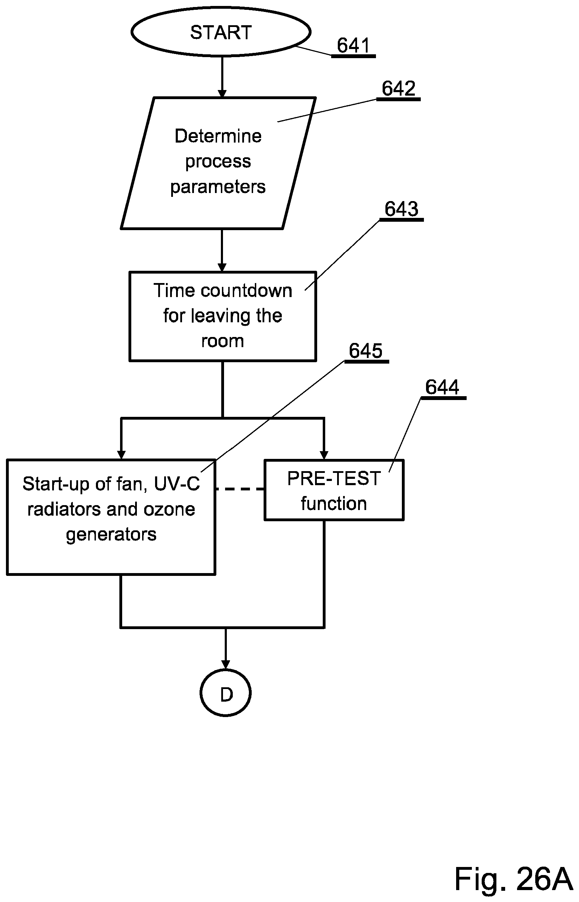

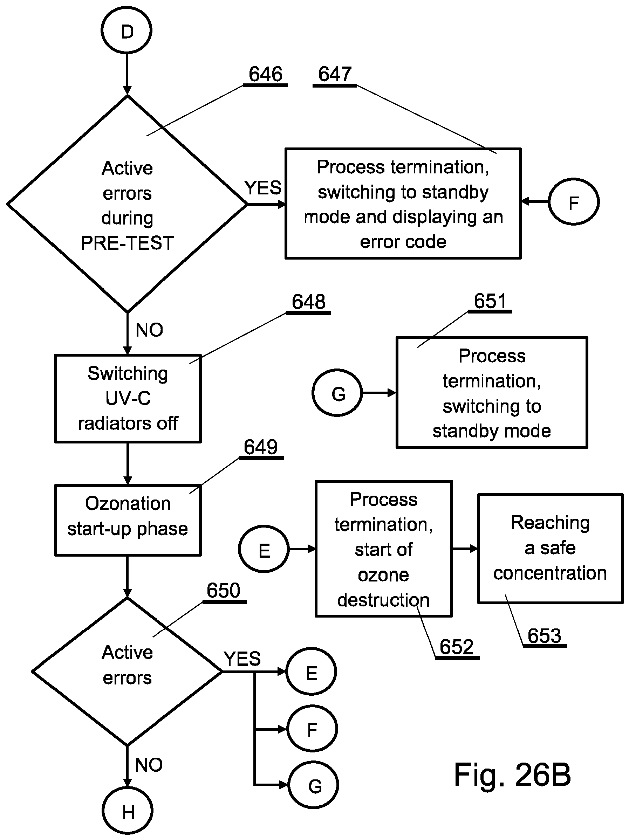

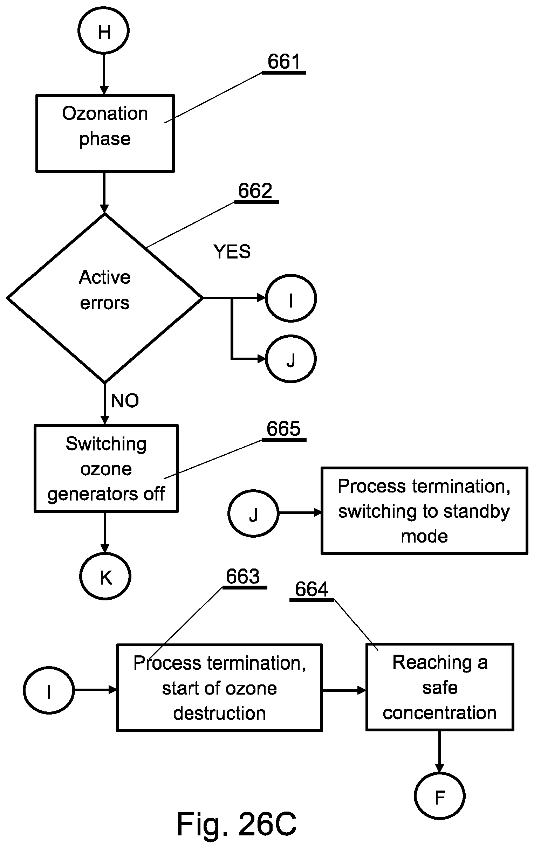

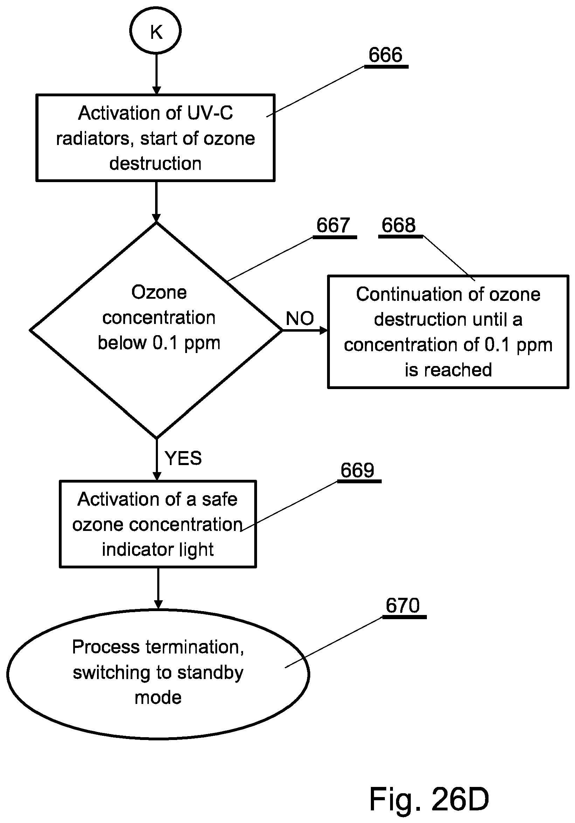

Fig. 26A, Fig. 26B, Fig. 26C and Fig. 26D show a block diagram of an algorithm of a sterilizer operation in an ozone sterilization mode. When the sterilizer is turned on in step 641, it is started. Then in step 642 process parameters, that is, operating time and operating concentration, are set manually. Once the parameters in step 643 have been set and accepted, the countdown to the start of the ozone generators begins. This is the time to leave the room safely. By default, this time is 120 seconds. After the time countdown, a PRE-TEST function in step 644 starts, during which fans, UV-C radiators and ozone generators are start-up or activated in step 645. If errors are detected in step 646, the process is terminated or aborted and follows switching to standby mode and corresponding error codes are displayed in step 647. If no errors are detected, the ozonation process starts and the radiators are switched off in step 648, as the light emitted by them would cause the ozone to decay more quickly. In step 649 the ozonation start-up phase takes place, in which the ozone concentration builds up until the working concentration is reached, and in step 650 the correct functioning of the sterilizer components is continuously monitored. If errors are detected in the ozone sensor, fan, or ozone generators, the process is terminated and follows switching to standby mode 651 or the process is aborted, and displays an error code in step 647. In addition, other errors may be detected, such as non-achievement of the required ozone concentration within a predetermined period of time or exceeding the ozone concentration limit. If they are detected, the procedure is similar, that is, the process terminates, returns to standby mode, an error code is displayed in step 647, or the process terminates and returns to standby mode in step 670. In addition, if the ozone concentration exceeds the allowable concentration, an ozone destruction process takes place in step 652 that continues until the safe concentration is reached in step 653.

After the start-up phase, an ozone phase starts in step 661. As with the start-up phase, in step 662, both errors of actuators, such as ozone sensors, ozone generators, fans, and process errors, for example exceeding the allowable ozone concentration, may be detected. If errors are detected, the process is terminated and the ozone is destroyed in step 663 until the safe concentration is reached in step 664, the process returns then to standby mode, and an error code is displayed in step 647. Once the ozonation process is terminated, the ozone generators are turned off in step 665, and in step 666 the UV-C radiators are activated and the ozone is destroyed until the safe concentration is reached in step 668, the level of which is checked in step 667. When the safe concentration of 0.1 ppm is reached, a green safe concentration light is activated in step 669. The process is terminated and the machine enters standby mode in step 670.

A particular advantage of the embodiments described here is that they are simple and compact, which makes it possible to adapt the size and performance of the sterilizers and, therefore, to be used in rooms of different sizes. In addition, the advantage is that the sterilizer can be selected according to the size of the rooms and the filtration, volatile medium disinfection and room ozonation modes can be set adequately to the needs, which ensures sterilization of the room.

The embodiments described above are only intended to describe the preferred embodiments of the present invention and are not intended to limit the scope of the present invention. Those skilled in the art can make the technical solutions of the present invention without departing from the scope of the present invention. as defined by the claims.

Various modifications and improvements are intended to fall within the scope of the invention as set out in the appended claims.

### List of references

- 1, 101, 201, 301, 401, 501, 502, 503, 504: Sterilizer
- 2, 102: Base
- 3, 103, 203, 303: Indicator
- 4, 104: Control panel
- 5: Volatile medium before purification
- 9: Volatile medium after purification
- 10, 110, 210, 310, 410, 510: Housing of sterilizer
- 11, 111, 211, 311: Inlet opening of filtered and disinfected volatile medium
- 12, 112: Outlet opening of filtered and disinfected volatile medium
- 13: Cover
- 14: Front wall
- 16: Grille 16
- 17: Pre-filter
- 18: Outlet filter
- 19: Outlet grille
- 20: Supply system
- 30: Forcing device of volatile medium flow
- 40: Control system
- 41: Switching unit of device for forcing volatile medium flow
- 44: Switching unit of ozone generators
- 47: Switching unit of UV-C radiators
- 48: Controller
- 49: Block of sensors
- 50: Disinfecting space
- 55: Inlet channel
- 60: Ozonation channel
- 61: Ozonation chamber
- 62: Ozone generator
- 65: Additional wall
- 66: Universal post
- 67: Side rail
- 68: Closing wall
- 70: Exposure channel
- 71: Exposure chamber
- 72: UV-C radiator
- 73: Air chamber
- 74: Upper filter chamber
- 75: Separating wall
- 76: Upper through hole
- 77: Through hole
- 78: Bend of separating wall
- 79: Bottom wall of housing of sterilizer
- 90: User panel
- 91: Main switch
- 92, 93, 94, 95, 96, 99: Function button
- 97: LEDs for warning, failure and safe ozone concentration
- 98: Digital display
- 162, 262: Set of ozone generators
- 169: Mounting hole
- 173: Socket
- 174: Bracket of socket
- 272: Set of double UV-C radiators
- 451: Stationary central unit
- 452: Pull-off duct
- 453: Blowing duct
- 459: Diffuser
- 490: Movable arm
- 491: Tube
- 492: Articulated joint
- 493: Inlet grille of volatile medium
- 495: Set of tubes
- 496: Stub pipe
- 499, 599: Interchangeable tip
- 500: System of simultaneous operation of multiple sterilizers
- 512: Spigot stub
- 549: Ozone sensor
- 595: Flexible pipe
- BANK 1, BANK 2: Bank of ozone generators

### Steps

- 601: START
- 602: PRE-TEST function
- 603: Start-up of fan
- 604: Active errors during PRE-TEST
- 605: Process termination, switching to standby mode and displaying an error code
- 606: Filtration by means of a standard filter
- 607: Filtration by means of an active carbon filter
- 608: Active errors during filtration
- 609: Process termination, switching to standby mode and displaying an error code
- 610: Process continuation
- 611: Process termination and return to standby mode
- 621: START
- 622: PRE-TEST function
- 623: Start-up of fan and UV-C radiators
- 624: Active errors during PRE-TEST
- 625: Process termination, switching to standby mode and displaying an error code
- 626: Filtration by means of a standard filter
- 627: Filtration by means of an active carbon filter
- 628: UV-C disinfection
- 629: Active errors
- 630: Process termination, switching to standby mode and displaying an error code
- 631: Process continuation
- 632: Process termination and return to standby mode
- 641: START
- 642: Determine process parameter
- 643: Time countdown for leaving the room
- 644: PRE-TEST function
- 645: Start-up of fan, UV-C radiators and ozone generators
- 646: Active errors during PRE-TEST
- 647: Process termination, switching to standby mode and displaying an error code
- 648: Switching UV-C radiators off
- 649: Ozonation start-up phase
- 650: Active errors
- 651: Process termination, switching to standby mode
- 652: Process termination, start of ozone destruction
- 653: Reaching a safe concentration
- 661: Ozonation phase
- 662: Active errors
- 663: Process termination, start of ozone destruction
- 664: Reaching a safe concentration
- 665: Switching ozone generators off
- 666: Activation of UV-C radiators, start of ozone destruction
- 667: Ozone concentration below 0.1 ppm
- 668: Continuation of ozone destruction until a concentration of 0.1 ppm is reached
- 669: Activation of a safe ozone concentration indicator light
- 670: Process termination, switching to standby mode

## Claims

1. A sterilizer (1, 101, 201, 301, 401, 501) for filtration and disinfection of volatile medium (5) comprising a disinfecting space (50) for filtration and disinfection of the volatile medium, through which the volatile medium (5) flows and which is surrounded by a housing (10), in which there is an inlet (11) and an outlet (12) of the filtered and disinfected volatile medium (5) connected to the disinfecting space (50), and a supply system (20), a forcing device (30) of volatile medium flow and a control system (40) for controlling the filtration and disinfection of the volatile medium flowing through the filtration and disinfection space of the volatile medium, wherein in the disinfection space (50) of the volatile medium there is at least one ozonation channel (60), each with at least one ozonation chamber (61) with at least one ozone generator (62), and at least one exposure channel (70), each with at least one exposure chamber (71) with at least one UV-C radiator (72), whereas a cross-sectional area of the ozonation channel (60) ranges from 250 cm² to 500 cm² and a cross-sectional area of the exposure channel (70) situated downstream of the ozonation channel (60) in the flow direction of the volatile medium (5) is from 250 cm² up to 600 cm², whereas the control system (40) for controlling the filtration and disinfection of the volatile medium comprises a unit (41) for switching-on and switching-off the device for forcing the volatile medium flow, a switching-on and switching-off unit (44) of the ozone generators (62) and a switching-on and switching-off unit (47) of the UV-C radiators (72) wherein the unit (41) for switching-on and switching-off the device for forcing the volatile medium flow, the switching-on and switching-off unit (44) of the ozone generators (62) and the switching-on and switching-off unit (47) of the UV-C radiators (72) operate independently of each other **characterized in that** adjacent to an outer wall of the sterilizer (1, 101, 201, 301, 401, 501) there is an inlet channel (55) that has a volatile medium inlet and is situated adjacent to the ozonation channel (60) and separated from the ozonation channel (60) by an additional wall (65) extending the entire width of an interior of the disinfection space (50), wherein the inlet channel (55) is connected to the ozonation channel (60) via a hole (76) located above the ozone generator (62) being the uppermost one and allowing the flow of the medium (5) from the inlet channel to the ozonation channel (60).

2. The sterilizer (1, 101, 201, 301, 401, 501) according to claim 1, **characterized in that** the hole (76) located above the uppermost placed ozone generator (62) and allowing the flow of volatile medium (5) from the inlet channel to the ozonation channel (60) is made in the additional wall (65) or is formed by positioning of an upper edge of the additional wall (65) below a closing wall (68) that closes the inlet channel (55) and the ozonation channel (60) from above.

3. The sterilizer (1, 101, 201, 301, 401, 501) according to claim 1 or 2, **characterized in that** the exposure channel (70) extends across the entire width of the interior of the disinfection space (50) and is situated adjacent to the ozonation channel (60) and separated from the ozonation channel (60) by a separating wall (75) and whereby the exposure channel (70) is connected to the ozonation channel (60) via a hole (77) located in a region of a bottom wall (79) of the sterilizer (1, 101, 201, 301, 401, 501) and allowing the flow of the volatile medium (5) from the ozonation channel (60) to the exposure channel.

4. The sterilizer (1, 101, 201, 301, 401, 501) according to claim 3, **characterized in that** the opening (77) located in the region of the bottom wall (79) of the sterilizer (1, 101, 201, 301, 401, 501) and allowing the flow of the volatile medium (5) from the ozonation channel (60) to the exposure channel (70) is made in the separating wall (75) or is formed by positioning a lower edge of the separating wall (75) above the bottom wall (79) which closes the ozonation channel (60) and the exposure channel (70) from below.

5. The sterilizer (1, 101, 201, 301, 401, 501) according to claim 1 or 2 or 3 or 4, **characterized in that** at side walls of the sterilizer (1, 101, 201, 301, 401, 501) there are situated posts (66) with holes for mounting the ozone generators (62) and limiting at least on sides the ozonation channel (60).

6. The sterilizer (1, 101, 201, 301, 401, 501) according to claim 1 or 2 or 3 or 4 or 5, **characterized in that** in a region of a lower part of the exposure channel (70) and in a region of an upper part of the exposure channel (70) there are sockets (173) for embedding the UV-C radiators (72).

7. The sterilizer (1, 101, 201, 301, 401, 501) according to claim 1 or 2 or 3 or 4 or 5 or 6, **characterized in that** the exposure channel (70) is connected to an air chamber (73) in which there is the device (30) forcing the flow of the volatile medium through the sterilizer (1, 101, 201, 301, 401, 501) and which is situated adjacent to an upper filter chamber (74) and an outlet of which is the outlet (12) of the filtered and disinfected volatile medium (5) covered by an outlet filter (18) and an outlet grille (19) or a spigot stub (512).

8. The sterilizer (401) according to claim 1 or 2 or 3 or 4 or 5 or 6, **characterized in that** the ozonation channel (60) is connected to the inlet of the filtered and disinfected volatile medium (5), situated at a front, side or back of the sterilizer and covered by a stub pipe (496), to which an additional movable arm (490) is mounted comprising a set (495) of tubes (491) connected to each other by articulated joints (492) and terminated with an interchangeable tip (499) for pulling off the filtered and disinfected volatile medium (5).

9. The sterilizer (401) according to claim 1 or 2 or 3 or 4 or 5 or 6, **characterized in that** the sterilizer is a stationary central unit (451) connected by at least one of its inlets to pull-off ducts (452) with interchangeable tips (499) for suction of the volatile mediumfrom one or more rooms and connected by at least one of its outlets to blowing ducts (453) with diffusers (459) for supplying purified volatile medium or ozone to one or more rooms.

10. The sterilizer (501) according to claim 7, **characterized in that** a flexible pipe (595) is connected to the spigot stub (512) having an interchangeable tip (599) placed inside a vehicle and used for supplying purified volatile medium or ozone to a vehicle interior.

11. The sterilizer (1, 101, 201, 301, 401) according to claim 1 or 2 or 3 or 4 or 5 or 6, **characterized in that** the sterilizer is one of sterilizers (501, 502, 503, 504) of a system (500) of simultaneous operating numerous sterilizers (501, 502, 503, 504) for filtration and disinfection of the volatile medium and for surface disinfection in enclosed spaces communicating by means of a control system to each sterilizer (501, 502, 503, 504) being associated with at least one sterilizer closest to it.

12. A method for filtration and disinfection of volatile medium in enclosed spaces using a sterilizer (1, 101, 201, 301, 401, 501), according to one of claims 1 to 11, for filtration and disinfection of volatile medium (5) and comprising a disinfection space (50) for filtration and disinfection of the volatile medium, through which the volatile medium (5) flows and which is surrounded by a housing (10), in which there is an inlet (11) and an outlet (12) of the filtered and disinfected volatile medium (5) connected to the disinfecting space (50), and a supply system (20), a forcing device (30) of volatile medium flow and a control system (40) for controlling the filtration and disinfection of the volatile medium flowing through the filtration and disinfection space of the volatile medium, wherein in the disinfection space (50) of the volatile medium there is at least one ozonation channel (60), each with at least one ozonation chamber (61) with at least one ozone generator (62), and at least one exposure channel (70), each with at least one exposure chamber (71) with at least one UV-C radiator (72), whereas a process selected from a volatile medium filtration process and/or a volatile medium disinfection process is carried out by means of the sterilizer (1, 101, 201, 301, 401, 501) for filtration and disinfection of the volatile medium (5) that has a cross-sectional area of the ozonation channel (60) ranging from 250 cm² to 500 cm² and the cross-sectional area of the exposure channel (70) situated downstream of the ozonation channel (60) in the flow direction of the volatile medium (5) ranging from 250 cm² up to 600 cm², and has the control system (40) for controlling the filtration and disinfection of the volatile medium which comprises a unit (41) for switching-on and switching-off the device for forcing the volatile medium flow, a switching-on and switching-off unit (44) of the ozone generators (62) and a switching-on and switching-off unit (47) of the UV-C radiators (72), wherein the unit (41) for switching-on and switching-off the device for forcing the volatile medium flow, the switching-on and switching-off unit (44) of the ozone generators (62) and the switching-on and switching-off unit (47) of the UV-C radiators (72) operate independently of each other and the volatile medium filtration process and/or the volatile medium disinfection process is selected by the control system (40) **characterized in that** adjacent to an outer wall of the sterilizer (1, 101, 201, 301, 401, 501) there is an inlet channel (55) that has a volatile medium inlet and is situated adjacent to the ozonation channel (60) and separated from the ozonation channel (60) by an additional wall (65) extending the entire width of an interior of the disinfection space (50), wherein the inlet channel (55) is connected to the ozonation channel (60) via a hole (76) located above the ozone generator (62) being the uppermost one and allowing the flow of the medium (5) from the inlet channel to the ozonation channel (60).

13. The method for filtration and disinfection of the volatile medium according to claim 12, **characterized in that** a process of ozone destruction to molecular oxygen begins after exceeding a predetermined level of ozone concentration in the volatile medium by switching on the UV-C radiators and by switched-off ozone generators and is carried until the safe ozone level is reached.

## Patentansprüche

1. Sterilisator (1, 101, 201, 301, 401, 501) zur Filtration und Desinfektion von flüchtigem Medium (5), umfassend einen Desinfektionsraum (50) zur Filtration und Desinfektion des flüchtigen Mediums, durch welchen das flüchtige Medium (5) strömt, und welcher von einem Gehäuse (10) umgeben ist, in welchem ein Einlass (11) und ein Auslass (12) des gefilterten und desinfizierten flüchtigen Mediums (5) sind, die mit dem Desinfektionsraum (50) verbunden sind, und ein Versorgungssystem (20), eine Vortriebsvorrichtung (30) der Strömung des flüchtigen Mediums und ein Steuersystem (40) zum Steuern der Filtration und Desinfektion des flüchtigen Mediums, das durch den Filtrations- und Desinfektionsraum des flüchtigen Mediums strömt, wobei sich im Desinfektionsraum (50) des flüchtigen Mediums zumindest ein Ozonisierungskanal (60) mit jeweils zumindest einer Ozonisierungskammer (61) mit zumindest einem Ozongenerator (62) und zumindest ein Expositionskanal (70) mit jeweils zumindest einer Expositionskammer (71) mit zumindest einem UV-C-Strahler (72) befindet, während eine Querschnittsfläche des Ozonisierungskanals (60) von 250 cm² bis 500 cm² reicht und eine Querschnittsfläche des Expositionskanals (70), der sich in Strömungsrichtung des flüchtigen Mediums (5) hinter dem Ozonisierungskanal (60) befindet, beträgt von 250 cm² bis 600 cm², während das Steuersystem (40) zum Steuern der Filtration und Desinfektion des flüchtigen Mediums eine Einheit (41) zum Ein- und Ausschalten der Vorrichtung zum Vortreiben der Strömung des flüchtigen Mediums, eine Ein- und Ausschalteinheit (44) der Ozongeneratoren (62) und eine Ein- und Ausschalteinheit (47) der UV-C-Strahler (72) umfasst, wobei die Einheit (41) zum Ein- und Ausschalten der Vorrichtung zum Vortreiben der Strömung des flüchtigen Mediums, die Ein- und Ausschalteinheit (44) der Ozongeneratoren (62) und die Ein- und Ausschalteinheit (47) der UV-C-Strahler (72) unabhängig voneinander arbeiten, **dadurch gekennzeichnet, dass** benachbart zu einer Außenwand des Sterilisators (1, 101, 201, 301, 401, 501) ein Einlasskanal (55) ist, der einen Einlass für flüchtige Medien aufweist und sich benachbart zu dem Ozonisierungskanal (60) befindet und von dem Ozonisierungskanal (60) durch eine zusätzliche Wand (65) getrennt ist, die sich über die gesamte Breite eines Innenraums des Desinfektionsraums (50) erstreckt, wobei der Einlasskanal (55) über ein Loch (76), das sich oberhalb des obersten Ozongenerator (62) befindet, mit dem Ozonisierungskanal (60) verbunden ist und die Strömung des Mediums (5) vom Einlasskanal zum Ozonisierungskanal (60) ermöglicht.

2. Sterilisator (1, 101, 201, 301, 401, 501) gemäß Anspruch 1, **dadurch gekennzeichnet, dass** das Loch (76), das sich über dem obersten platzierten Ozongenerator (62) befindet und den Fluss von flüchtigem Medium (5) von dem Einlasskanal zu dem Ozonisierungskanal (60) ermöglicht, in der zusätzlichen Wand (65) hergestellt ist oder durch Positionieren einer oberen Kante der zusätzlichen Wand (65) unter einer Verschlusswand (68) gebildet ist, die den Einlasskanal (55) und den Ozonisierungskanal (60) von oben verschließt.

3. Sterilisator (1, 101, 201, 301, 401, 501) gemäß Anspruch 1 oder 2, **dadurch gekennzeichnet, dass** sich der Expositionskanal (70) über die gesamte Breite des Innenraums des Desinfektionsraums (50) erstreckt und sich benachbart zu dem Ozonisierungskanal (60) befindet und von dem Ozonisierungskanal (60) durch eine Trennwand (75) getrennt ist und wobei der Expositionskanal (70) mit dem Ozonisierungskanal (60) über eine Öffnung (77) verbunden ist, das sich in einer Region einer Bodenwand (79) des Sterilisators (1, 101, 201, 301, 401, 501) befindet und die Strömung des flüchtigen Mediums (5) von dem Ozonisierungskanal (60) zu dem Expositionskanal ermöglicht.

4. Sterilisator (1, 101, 201, 301, 401, 501) gemäß Anspruch 3, **dadurch gekennzeichnet, dass** die Öffnung (77), die sich in der Region der Bodenwand (79) des Sterilisators (1, 101, 201, 301, 401, 501) befindet und die Strömung des flüchtigen Mediums (5) von dem Ozonisierungskanal (60) zu dem Expositionskanal (70) ermöglicht, in der Trennwand (75) hergestellt ist oder durch die Positionierung einer unteren Kante der Trennwand (75) über der Bodenwand (79) gebildet ist, welche den Ozonisierungskanal (60) und den Expositionskanal (70) von unten verschließt.

5. Sterilisator (1, 101, 201, 301, 401, 501) gemäß Anspruch 1 oder 2 oder 3 oder 4, **dadurch gekennzeichnet, dass** sich an Seitenwänden des Sterilisators (1, 101, 201, 301, 401, 501) Pfosten (66) mit Löchern zum Montieren der Ozongeneratoren (62) befinden und zumindest an Seiten den Ozonisierungskanal (60) begrenzen.

6. Sterilisator (1, 101, 201, 301, 401, 501) gemäß Anspruch 1 oder 2 oder 3 oder 4 oder 5, **dadurch gekennzeichnet, dass** es in einer Region eines unteren Teils des Expositionskanals (70) und in einer Region eines oberen Teils des Expositionskanals (70) Steckdosen (173) zum Einbetten der UV-C-Strahler (72) gibt.

7. Sterilisator (1, 101, 201, 301, 401, 501) gemäß Anspruch 1 oder 2 oder 3 oder 4 oder 5 oder 6, **dadurch gekennzeichnet, dass** der Expositionskanal (70) mit einer Luftkammer (73) verbunden ist, in welcher die Vorrichtung (30) ist, die die Strömung des flüchtigen Mediums durch den Sterilisator (1, 101, 201, 301, 401, 501) vortreibt und welche benachbart zu einer oberen Filterkammer (74) und einem Auslass ist, der der Auslass (12) des gefilterten und desinfizierten flüchtigen Mediums (5) ist, das durch einen Auslassfilter (18) und ein Auslassgitter (19) oder einen Zapfenstumpf (512) abgedeckt ist.

8. Sterilisator (401) gemäß Anspruch 1 oder 2 oder 3 oder 4 oder 5 oder 6, **dadurch gekennzeichnet, dass** der Ozonisierungskanal (60) mit dem Einlass des gefilterten und desinfizierten flüchtigen Mediums (5) verbunden ist, der sich an einer Vorderseite, Seite oder Rückseite des Sterilisators befindet und durch ein Stumpfrohr (496) abgedeckt ist, an welchem ein zusätzlicher beweglicher Arm (490) montiert ist, der einen Satz (495) von Röhren (491) umfasst, die durch Gelenkverbindungen (492) miteinander verbunden sind und mit einer austauschbaren Spitze (499) zum Abziehen des gefilterten und desinfizierten flüchtigen Mediums (5) enden.

9. Sterilisator (401) gemäß Anspruch 1 oder 2 oder 3 oder 4 oder 5 oder 6, **dadurch gekennzeichnet, dass** der Sterilisator eine stationäre Zentraleinheit (451) ist, die durch zumindest einen ihrer Einlässe mit Abziehdurchgängen (452) mit austauschbaren Spitzen (499) zum Absaugen des flüchtigen Mediums aus einem oder mehreren Räumen verbunden ist und durch zumindest einen ihrer Auslässe mit Blasdurchgängen (453) mit Diffusoren (459) zum Zuführen von gereinigtem flüchtigen Medium oder Ozon zu einem oder mehreren Räumen verbunden ist.

10. Sterilisator (501) gemäß Anspruch 7, **dadurch gekennzeichnet, dass** ein flexibles Rohr (595) mit dem Zapfenstumpf (512) verbunden ist, der eine austauschbare Spitze (599) aufweist, die in einem Fahrzeug platziert ist und zum Zuführen von gereinigtem flüchtigem Medium oder Ozon zu einem Fahrzeuginnenraum verwendet wird.

11. Sterilisator (1, 101, 201, 301, 401) gemäß Anspruch 1 oder 2 oder 3 oder 4 oder 5 oder 6, **dadurch gekennzeichnet, dass** der Sterilisator einer der Sterilisatoren (501, 502, 503, 504) eines Systems (500) des gleichzeitigen Betriebs mehrerer Sterilisatoren (501, 502, 503, 504) zur Filtration und Desinfektion des flüchtigen Mediums und zur Oberflächendesinfektion in geschlossenen Räumen ist, die mittels eines Steuersystems mit jedem Sterilisator (501, 502, 503, 504) kommunizieren, wobei sie zumindest einem Sterilisator zugeordnet, der ihm am nächsten ist.

12. Verfahren zur Filtration und Desinfektion von flüchtigem Medium in geschlossenen Räumen unter Verwendung eines Sterilisators (1, 101, 201, 301, 401, 501) gemäß einem der Ansprüche 1 bis 11 zur Filtration und Desinfektion von flüchtigem Medium (5) und umfassend einen Desinfektionsraum (50) zur Filtration und Desinfektion des flüchtigen Mediums, durch welchen das flüchtige Medium (5) strömt und welcher von einem Gehäuse (10) umgeben ist, in welchem ein Einlass (11) und ein Auslass (12) des gefilterten und desinfizierten flüchtigen Mediums (5) sind, das mit dem Desinfektionsraum (50) und einem Versorgungssystem (20), einer Vortriebsvorrichtung (30) für die Strömung des flüchtigen Mediums und einem Steuersystem (40) zum Steuern der Filtration und Desinfektion des flüchtigen Mediums verbunden ist, das durch den Filtrations- und Desinfektionsraum des flüchtigen Mediums strömt, wobei in dem Desinfektionsraum (50) des flüchtigen Mediums zumindest ein Ozonisierungskanal (60) mit jeweils zumindest einer Ozonisierungskammer (61) mit zumindest einem Ozongenerator (62) und zumindest einem Expositionskanal (70) ist, jeder mit zumindest einer Expositionskammer (71) mit zumindest einem UV-C-Strahler (72), während mittels des Sterilisators (1, 101, 201, 301, 401, 501) ein Prozess, der ausgewählt ist aus einem Filtrationsprozess eines flüchtigen Mediums und/oder Desinfektionsprozess eines flüchtigen Mediums, zur Filtration und Desinfektion des flüchtigen Mediums (5) ausgeführt wird, der eine Querschnittsfläche des Ozonisierungskanals (60) im Bereich von 250 cm² bis 500 cm² aufweist und der Querschnittsbereich des Expositionskanals (70), der sich in Strömungsrichtung des flüchtigen Mediums (5) hinter dem Ozonisierungskanal (60) befindet, reicht von 250 cm² bis 600 cm² und weist das Steuersystem (40) zum Steuern der Filtration und Desinfektion des flüchtigen Mediums auf, das eine Einheit (41) zum Ein- und Ausschalten der Vorrichtung zum Vortreiben der Strömung des flüchtigen Mediums, eine Ein- und Ausschalteinheit (44) der Ozongeneratoren (62) und eine Einschalt- und Ausschalteinheit (47) der UV-C-Strahler (72) umfasst, wobei die Einheit (41) zum Ein- und Ausschalten der Vorrichtung zum Vortreiben der Strömung des flüchtigen Mediums, die Ein- und Ausschalteinheit (44) der Ozongeneratoren (62) und die Ein- und Ausschalteinheit (47) der UV-C-Strahler (72) unabhängig voneinander arbeiten und der Filtrationsprozess des flüchtigen Mediums und/oder der Desinfektionsprozess des flüchtigen Mediums durch das Steuersystem (40) ausgewählt wird, **dadurch gekennzeichnet, dass** benachbart zu einer Außenwand des Sterilisators (1, 101, 201, 301, 401, 501) ein Einlasskanal (55) ist, der einen Einlass für flüchtige Medien aufweist und sich benachbart zu dem Ozonisierungskanal (60) befindet und von dem Ozonisierungskanal (60) durch eine zusätzliche Wand (65) getrennt ist, die sich über die gesamte Breite eines Innenraums des Desinfektionsraums (50) erstreckt, wobei der Einlasskanal (55) mit dem Ozonisierungskanal (60) über ein Loch (76) verbunden ist, das sich oberhalb des Ozongenerators (62) befindet, der der oberste ist und die Strömung des Mediums (5) von dem Einlasskanal zu dem Ozonisierungskanal (60) ermöglicht.

13. Verfahren zur Filtration und Desinfektion des flüchtigen Mediums gemäß Anspruch 12, **dadurch gekennzeichnet, dass** ein Prozess der Ozonzerstörung zu molekularem Sauerstoff nach Überschreiten eines vorbestimmten Niveaus der Ozonkonzentration in dem flüchtigen Medium durch Einschalten der UV-C-Strahler und durch Ausschalten der Ozongeneratoren beginnt und bis zum Erreichen des sicheren Ozonniveaus ausgeführt wird.

## Revendications

1. Stérilisateur (1, 101, 201, 301, 401, 501) destiné à la filtration et la désinfection d'une substance volatile (5) comprenant un espace de désinfection (50) destiné à la filtration et la désinfection de la substance volatile, à travers lequel s'écoule la substance volatile (5) et qui est entouré par un boîtier (10), dans lequel se trouvent une entrée (11) et une sortie (12) de la substance volatile filtrée et désinfectée (5) raccordées à l'espace de désinfection (50), et un système d'alimentation (20), un dispositif de forçage (30) de l'écoulement de la substance volatile et un système de commande (40) destiné à commander la filtration et la désinfection de la substance volatile s'écoulant à travers l'espace de filtration et de désinfection de la substance volatile, tandis que dans l'espace de désinfection (50) de la substance volatile se trouve au moins un canal d'ozonation (60), chacun doté d'au moins une chambre d'ozonation (61) dotée d'au moins un générateur d'ozone (62), et au moins un canal d'exposition (70), chacun doté d'au moins une chambre d'exposition (71) dotée d'au moins un émetteur de rayonnement UV-C (72), alors que la surface de section transversale du canal d'ozonation (60) va de 250 cm² à 500 cm² et la surface de section transversale du canal d'exposition (70) situé en aval du canal d'ozonation (60) dans le sens d'écoulement de la substance volatile (5) est de 250 cm² à 600 cm², tandis que le système de commande (40) destiné à commander la filtration et la désinfection de la substance volatile comprend une unité (41) destinée à mettre en marche et arrêter le dispositif destiné à forcer le flux de substance volatile, une unité de mise en marche et d'arrêt (44) des générateurs d'ozone (62) et une unité de mise en marche et d'arrêt (47) des émetteurs de rayonnement UV-C (72), ladite unité (41) destinée à mettre en marche et arrêter le dispositif destiné à forcer l'écoulement de la substance volatile, ladite unité de mise en marche et d'arrêt (44) des générateurs d'ozone (62) et ladite unité de mise en marche et d'arrêt (47) des émetteurs de rayonnement UV-C (72) fonctionnant indépendamment les unes des autres, **caractérisé en ce que**, à côté d'une paroi externe du stérilisateur (1, 101, 201, 301, 401, 501), se trouve un canal d'entrée (55) qui comporte une entrée de substance volatile et est situé à côté du canal d'ozonation (60) et séparé du canal d'ozonation (60) par une paroi supplémentaire (65) s'étendant sur toute la largeur de l'intérieur de l'espace de désinfection (50), ledit canal d'entrée (55) étant raccordé au canal d'ozonation (60) par l'intermédiaire d'un trou (76) situé au-dessus du générateur d'ozone (62) étant le plus haut et permettant l'écoulement de la substance (5) du canal d'entrée au canal d'ozonation (60).

2. Stérilisateur (1, 101, 201, 301, 401, 501) selon la revendication 1, **caractérisé en ce que** le trou (76) situé au-dessus du générateur d'ozone (62) placé le plus haut et permettant l'écoulement de la substance volatile (5) du canal d'entrée au canal d'ozonation (60) est réalisé dans la paroi supplémentaire (65) ou est formé par le positionnement d'un bord supérieur de la paroi supplémentaire (65) en dessous d'une paroi de fermeture (68) qui ferme le canal d'entrée (55) et le canal d'ozonation (60) par le haut.

3. Stérilisateur (1, 101, 201, 301, 401, 501) selon la revendication 1 ou 2, **caractérisé en ce que** le canal d'exposition (70) s'étend sur toute la largeur de l'intérieur de l'espace de désinfection (50) et est situé à côté du canal d'ozonation (60) et séparé du canal d'ozonation (60) par une paroi de séparation (75) tandis que le canal d'exposition (70) est raccordé au canal d'ozonation (60) par l'intermédiaire d'une ouverture (77) située dans une zone d'une paroi inférieure (79) du stérilisateur (1, 101, 201, 301, 401, 501) et permettant l'écoulement de la substance volatile (5) du canal d'ozonation (60) au canal d'exposition.

4. Stérilisateur (1, 101, 201, 301, 401, 501) selon la revendication 3, **caractérisé en ce que** l'ouverture (77) située dans la zone de la paroi inférieure (79) du stérilisateur (1, 101, 201, 301, 401, 501) et permettant l'écoulement de la substance volatile (5) du canal d'ozonation (60) au canal d'exposition (70) est réalisée dans la paroi de séparation (75) ou est formée par le positionnement d'un bord inférieur de la paroi de séparation (75) au-dessus de la paroi inférieure (79) qui ferme le canal d'ozonation (60) et le canal d'exposition (70) par le bas.

5. Stérilisateur (1, 101, 201, 301, 401, 501) selon la revendication 1 ou 2 ou 3 ou 4, **caractérisé en ce qu'**au niveau des parois latérales du stérilisateur (1, 101, 201, 301, 401, 501) se trouvent des montants (66) dotés de trous destinés à monter les générateurs d'ozone (62) et limiter au moins sur les côtés le canal d'ozonation (60).

6. Stérilisateur (1, 101, 201, 301, 401, 501) selon la revendication 1 ou 2 ou 3 ou 4 ou 5, **caractérisé en ce que** dans une zone d'une partie inférieure du canal d'exposition (70) et dans une zone d'une partie supérieure du canal d'exposition (70) se trouvent des prises (173) destinées à intégrer les émetteurs de rayonnement UV-C (72).

7. Stérilisateur (1, 101, 201, 301, 401, 501) selon la revendication 1 ou 2 ou 3 ou 4 ou 5 ou 6, **caractérisé en ce que** le canal d'exposition (70) est raccordé à une chambre à air (73) dans laquelle se trouve le dispositif (30) forçant l'écoulement de la substance volatile à travers le stérilisateur (1, 101, 201, 301, 401, 501) et qui est situé à côté d'une chambre de filtre supérieure (74) et dont une sortie est la sortie (12) de la substance volatile filtrée et désinfectée (5) recouverte par un filtre de sortie (18) et une grille de sortie (19) ou un embout mâle (512).

8. Stérilisateur (401) selon la revendication 1 ou 2 ou 3 ou 4 ou 5 ou 6, **caractérisé en ce que** le canal d'ozonation (60) est raccordé à l'entrée de la substance volatile filtrée et désinfectée (5), situé à l'avant, sur le côté ou à l'arrière du stérilisateur et recouvert d'une tubulure (496), sur laquelle est monté un bras mobile supplémentaire (490) comprenant un ensemble (495) de tubes (491) raccordés les uns aux autres par des joints articulés (492) et se terminant par une pointe interchangeable (499) destinée à retirer la substance volatile filtrée et désinfectée (5).

9. Stérilisateur (401) selon la revendication 1 ou 2 ou 3 ou 4 ou 5 ou 6, **caractérisé en ce que** le stérilisateur est une unité centrale fixe (451) raccordée par au moins l'une de ses entrées à des conduits d'extraction (452) dotés d'embouts interchangeables (499) destinés à aspirer la substance volatile d'une ou plusieurs pièces et raccordée par au moins l'une de ses sorties à des conduits de soufflage (453) dotés de diffuseurs (459) destinés à alimenter en substance volatile purifiée ou en ozone une ou plusieurs pièces.

10. Stérilisateur (501) selon la revendication 7, **caractérisé en ce qu'**un tuyau flexible (595) est raccordé à l'embout mâle (512) comportant une pointe interchangeable (599) placée à l'intérieur d'un véhicule et utilisé pour fournir de la substance volatile purifiée ou de l'ozone à l'intérieur d'un véhicule.

11. Stérilisateur (1, 101, 201, 301, 401) selon la revendication 1 ou 2 ou 3 ou 4 ou 5 ou 6, **caractérisé en ce que** le stérilisateur est l'un des stérilisateurs (501, 502, 503, 504) d'un système (500) de nombreux stérilisateurs (501, 502, 503, 504) fonctionnant simultanément pour la filtration et la désinfection de la substance volatile et pour la désinfection de surface dans des espaces clos communiquant au moyen d'un système de commande à chaque stérilisateur (501, 502, 503, 504) étant associés à au moins un stérilisateur le plus proche de lui.

12. Procédé de filtration et de désinfection d'une substance volatile dans des espaces clos à l'aide d'un stérilisateur (1, 101, 201, 301, 401, 501), selon l'une des revendications 1 à 11, destiné à la filtration et la désinfection de la substance volatile (5) et comprenant un espace de désinfection (50) destiné à la filtration et la désinfection de la substance volatile, à travers lequel s'écoule la substance volatile (5) et qui est entouré par un boîtier (10), dans lequel se trouvent une entrée (11) et une sortie (12) de la substance volatile filtrée et désinfectée (5) raccordé à l'espace de désinfection (50), et un système d'alimentation (20), un dispositif de forçage (30) de l'écoulement de la substance volatile et un système de commande (40) destiné à commander la filtration et la désinfection de la substance volatile s'écoulant à travers l'espace de filtration et de désinfection de la substance volatile, dans ledit espace de désinfection (50) de la substance volatile se trouvant au moins un canal d'ozonation (60), chacun doté d'au moins une chambre d'ozonation (61) dotée d'au moins un générateur d'ozone (62), et au moins un canal d'exposition (70), chacun doté d'au moins une chambre d'exposition (71) dotée d'au moins un émetteur de rayonnement UV-C (72), tandis qu'un processus sélectionné parmi un processus de filtration de substance volatile et/ou un processus de désinfection de substance volatile est effectué au moyen du stérilisateur (1, 101, 201, 301, 401, 501) destiné à la filtration et la désinfection de la substance volatile (5) qui possède une surface de section transversale du canal d'ozonation (60) allant de 250 cm² à 500 cm² et la zone de section transversale du canal d'exposition (70) situé en aval du canal d'ozonation (60) dans le sens d'écoulement de la substance volatile (5) allant de 250 cm² à 600 cm², et comporte le système de commande (40) destiné à commander la filtration et la désinfection de la substance volatile qui comprend une unité (41) destinée à la mise en marche et l'arrêt du dispositif destiné à forcer l'écoulement de la substance volatile, une unité de mise en marche et d'arrêt (44) des générateurs d'ozone (62) et une unité de mise en marche et d'arrêt (47) des émetteurs de rayonnement UV-C (72), ladite unité (41) destinée à la mise en marche et à l'arrêt du dispositif destiné à forcer l'écoulement de la substance volatile, l'unité de mise en marche et d'arrêt (44) des générateurs d'ozone (62) et l'unité de mise en marche et d'arrêt (47) des émetteurs de rayonnement UV-C (72) fonctionnant indépendamment l'une de l'autre et le processus de filtration de la substance volatile et/ou le processus de désinfection de la substance volatile étant sélectionné par le système de commande (40), **caractérisé en ce que**, à côté d'une paroi externe du stérilisateur (1, 101, 201, 301, 401, 501), se trouve un canal d'entrée (55) qui comporte une entrée de substance volatile et est situé à côté du canal d'ozonation (60) et séparé du canal d'ozonation (60) par une paroi supplémentaire (65) s'étendant sur toute la largeur de l'intérieur de l'espace de désinfection (50), ledit canal d'entrée (55) étant raccordé au canal d'ozonation (60) par l'intermédiaire d'un trou (76) situé au-dessus du générateur d'ozone (62) étant le plus haut et permettant l'écoulement de la substance (5) du canal d'entrée au canal d'ozonation (60).

13. Procédé de filtration et de désinfection de la substance volatile selon la revendication 12, **caractérisé en ce qu'**un processus de destruction de l'ozone en oxygène moléculaire commence après avoir dépassé un niveau prédéfini de concentration d'ozone dans la substance volatile en mettant en marche les émetteurs de rayonnement UV-C et en arrêtant les générateurs d'ozone et est effectué jusqu'à ce que le niveau d'ozone sûr soit atteint.
